# EUROPEAN PATENT APPLICATION

(11) **EP 2 226 325 A1**
(43) Date of publication of application: **08.09.2010**
(21) Application number: 08863919.0
(22) Date of filing: 25.12.2008
(51) Int. Cl.: C07D 498/04, C07D 265/30

(54) **PROCESS FOR PRODUCTION OF 4-(SUBSTITUTED PHENYL)HEXAHYDROPYRIDOÝ2,1-C¨Ý1,4¨OXAZIN-6-ONE**

(30) Priority: 26.12.2007 US 16796
(71) Applicant: Eisai R&D Management Co., Ltd., Tokyo 112-8088 (JP)
(72) Inventor: KAYANO, Akio, Kamisu-shi Ibaraki 314-0255 (JP); NAGAI, Mitsuo, Tsukuba-shi Ibaraki 300-2635 (JP); HOSHINO, Yorihisa, Tsukuba-shi Ibaraki 300-2635 (JP); WAKASUGI, Kazunori, Tsukuba-shi Ibaraki 300-2635 (JP); MATSUDA, Masaaki, Tsukuba-shi Ibaraki 300-2635 (JP); KAMADA, Atsushi, Tsukuba-shi Ibaraki 300-2635 (JP); ISOMURA, Minetaka, Kamisu-shi Ibaraki 314-0255 (JP); NISHIKAWA, Yoshihiro, Kamisu-shi Ibaraki 314-0255 (JP); YOSHIKAWA, Seiji, Kamisu-shi Ibaraki 314-0255 (JP); SHIMMYO, Daisuke, Bunkyo-ku, Tokyo 112-8088 (JP); DOI, Eriko, Tsukuba-shi Ibaraki 300-2635 (JP); KANEKO, Toshihiko, Bunkyo-ku, Tokyo 112-8088 (JP)
(74) Representative: HOFFMANN EITLE
(86) International application number: PCT/JP2008/073513
(87) International publication number: WO 2009/081959

(57) **Abstract**

4-(Substituted phenyl)hexahydropyrido[2,1-c][1,4]oxazin-6-one represented by formula (I) or a salt thereof is useful as an intermediate for the production of a bicyclic cinnamide compound which is an Aβ production inhibitor. 4-(Substituted phenyl)hexahydropyrido[2,1-c][1,4]oxazin-6-one or the salt thereof can be produced in an industrially advantageous manner by subjecting a compound represented by formula (II) or a salt thereof to an intramolecular condensation reaction.

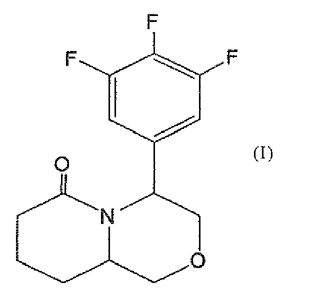

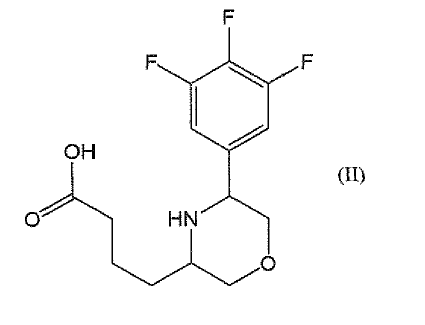

## Description

### TECHNICAL FIELD

The present invention relates to a process for production of 4-(substituted phenyl)hexahydropyrido[2,1-c][1,4]oxazin-6-one that is an intermediate product of a compound useful as an amyloid beta (hereinafter, referred to as Aβ) production inhibitor effective for treating neurodegenerative diseases such as Alzheimer's disease and Down syndrome which are caused by Aβ. More preferably, the present invention also relates to a new compound used in the production process.

### BACKGROUND ART

A bicyclic cinnamide compound represented by the following formula is known as an Aβ production inhibitor: 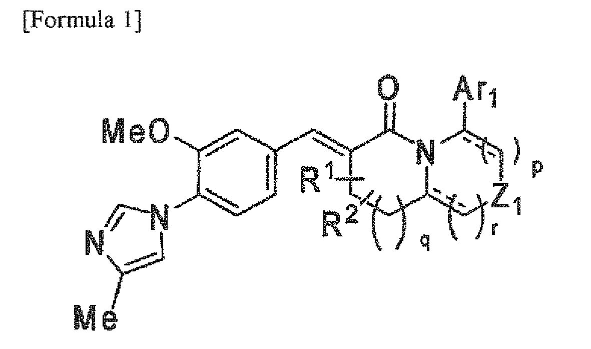 wherein - represents a single bond or a double bond; Ar₁ represents a phenyl group or a pyridinyl group which may be substituted with 1 to 3 substituents; R¹ and R² represent a C1-6 alkyl group, a hydroxyl group, and the like; Z₁ represents a methylene group or a vinylene group which may be substituted, an oxygen atom, or an imino group which may be substituted with a C1-6 alkyl group or a C1-6 acyl group; and p, q, and r represent an integer of 0 to 2] (PATENT DOCUMENT 1). PATENT DOCUMENT 1 discloses a 4-(substituted phenyl)hexahydropyrido[2,1-c][1,4]oxazin-6-one compound represented by a compound of the following formula (I) as an intermediate product of the Example compound:

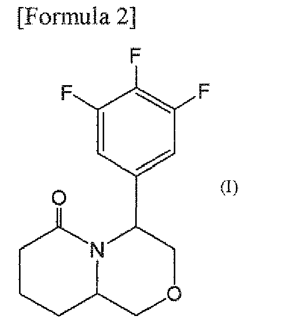

PATENT DOCUMENT 1: International Publication WO07/060821

### DISCLOSURE OF THE INVENTION

### PROBLEMS TO BE SOLVED BY THE INVENTION

However, a process for production of such compounds has not been necessarily a sufficient industrial production process because it uses expensive reagents, requires column purification and many steps so that the yield is not so high, and a load to the environment is not small.

### MEANS FOR SOLVING THE PROBLEMS

In such circumstances, the present inventors have conducted intensive studies and have found the present invention.

The present invention relates to the followings.
(1) A process for production of 4-(substituted phenyl)hexahydropyrido[2,1-c][1,4]oxazin-6-one or a salt thereof by subjecting a compound represented by the following formula (II-a): 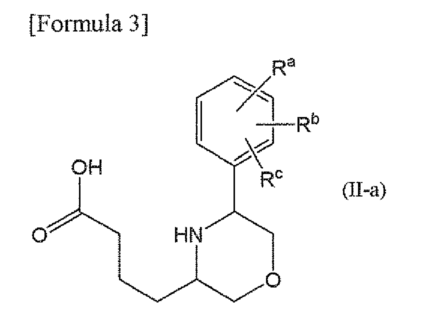 wherein R^{a}, R^{b} and R^{c} each independently represent a hydrogen atom, a halogen atom, a cyano group, an amino group, a nitro group, a C1-6 alkyl group or a C1-6 alkoxy group] or a salt thereof to an intramolecular condensation reaction, thereby producing a compound represented by the following formula (I-a): 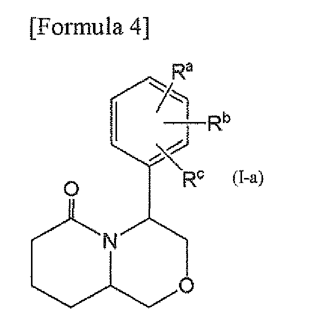 [wherein R^{a}, R^{b} and R^{c} are as defined above] or a salt thereof.
(2) The production process described in (I), wherein R^{a}, R^{b} and R^{c} are each independently a hydrogen atom or a halogen atom.
(3) A process for production of 4-(3,4,5-trifluorophenyl)hexahydropyrido[2,1-c][1,4]oxazin-6-one or a salt thereof by subjecting a compound represented by the following formula (II): 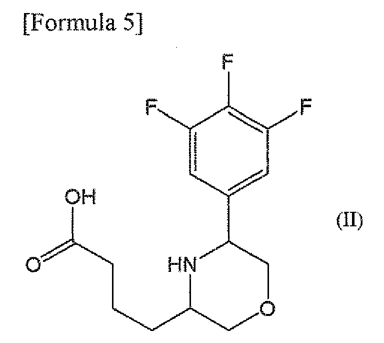 or a salt thereof to an intramolecular condensation reaction, thereby producing a compound represented by the following formula (I): 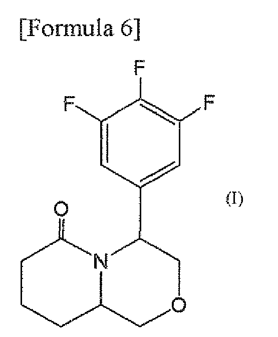 or a salt thereof.
(4) A process for production of (4S,9aR)-4-(3,4,5-trifluorophenyl)hexahydropyrido[2,1-c][1,4]oxazin-6-one or a salt thereof by subjecting a compound represented by the following formula (II-b): 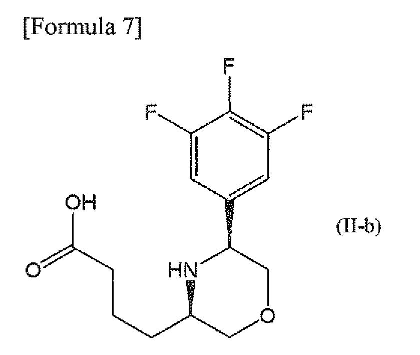 or a salt thereof to an intramolecular condensation reaction, thereby producing a compound represented by the following formula (I-b): 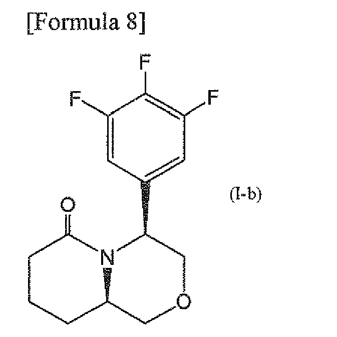 or a salt thereof.
(5) A process for production of (4R,9aS)-4-(3,4,5-trifluorophenyl) hexahydropyrido[2,1-c][1,4]oxazin-6-one or a salt thereof by subjecting a compound represented by a formula (II-c): 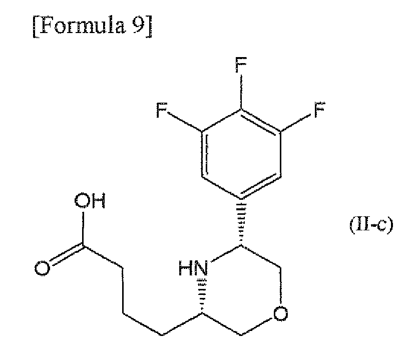 or a salt thereof to an intramolecular condensation reaction, thereby producing a compound represented by the following formula (Y-c): 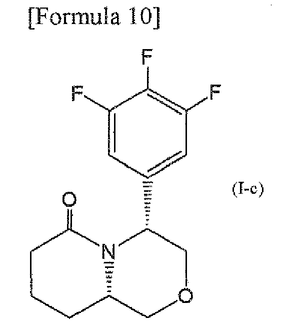 or a salt thereof.
(6) A compound represented by the following formula (II-a): 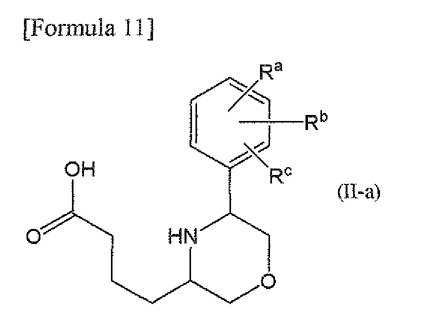 wherein R^{a}, R^{b} and R^{c} each independently represent a hydrogen atom, a halogen atom, a cyano group, an amino group, a nitro group, a C1-6 alkyl group or a C1-6 alkoxy group] or a salt thereof.
(7) A compound represented by the following formula (II): 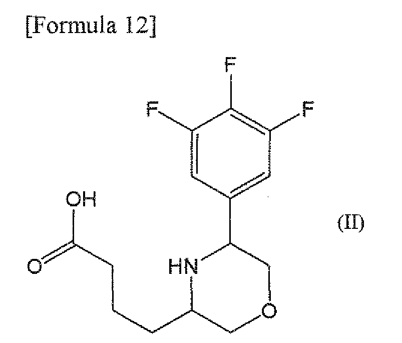 or a salt thereof.
(8) A compound represented by the following formula (II-b): 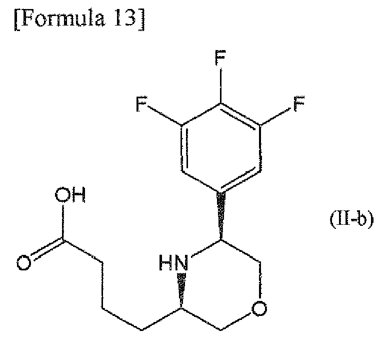 or a salt thereof.
(9) A compound represented by the following formula (II-c): 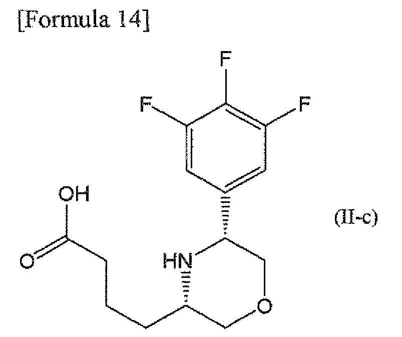 or a salt thereof.

### ADVANTAGES OF THE INVENTION

The present invention makes it possible to produce 4-(substituted phenyl)hexahydropyrido[2,1-c][1,4]oxazin-6-one useful as an intermediate product of a bicyclic cinnamide compound that is an Aβ production inhibitor industrially advantageously. Furthermore, the present invention also provides a new compound used in the production.

### BEST MODE FOR CARRYING OUT THE INVENTION

Hereinafter, a production process of the present invention is described in detail.
In the above-mentioned formulae: (II-a) and (I-a) according to the production process of the present invention, the terms "halogen atom "C1-6 alkyl group" and "C1-6 alkoxy group" have the following meanings.

The term "halogen atom" represents a fluorine atom, a chlorine atom, a bromine atom, an iodine atom, and the like, and preferably a fluorine atom, a chlorine atom, or a bromine atom.

The term "C1-6 alkyl group" represents an alkyl group having 1 to 6 carbon atoms. Preferable examples of the groups include linear or branched alkyl groups, for example, a methyl group, an ethyl group, a n-propyl group, an i-propyl group, a n-butyl group, an i-butyl group, a tert-butyl group, a n-pentyl group, an i-pentyl group, a neopentyl group, a n-hexyl group, a 1-methylpropyl group, a 1,2-dirnethylprapyl group, an 1-ethylpropyl group, a 1-methyl-2-ethylpropyl group, an 1-ethyl-2-methylpropyl group, a 1,1,2-trimethylpropyl group, a 1-methylbutyl group, a 2-methylbutyl group, a 1,1-dimethylbutyl group, a 2,2-dimethylbutyl group, a 2-ethylbutyl group, a 1,3-dimethylbutyl group, a 2-methylpentyl group, a 3-methylpentyl group, and the like.

The term "C1-6 alkoxy group" represents a group in which an alkyl group having 1 to 6 carbon atoms is substituted with an oxygen atom. Preferable examples of the group include a methoxy group, an ethoxy group, a n-propoxy group, an i-propoxy group, a n-butoxy group, an i-butoxy group, a sec-butoxy group, a tert-butoxy group, a n-pentyloxy group, an i-pentyloxy group, a sec-pentyloxy group, a tert-pentyloxy group, a n-hexyloxy group, an i-hexyloxy group, a 1,2-dimethylpropoxy group, an 2-ethylpropoxy group, a 1-methyl-2-ethylpropoxy group, an 1-ethyl-2-methylpropoxy group, a 1,1,2-trimethylpropoxy group, a 1,1,2-trimethylpropoxy group, a 1,1-dimethylbutoxy group, a 2,2-dimethylbutoxy group, an 2-ethylbutoxy group, a 1,3-dimethylbutoxy group, a 2-methylpentyloxy group, a 3-methylpentyloxy group, a hexyloxy group, and the like.

The intramolecular condensation reaction in the production process of the present invention can be carried out in a flow or an atmosphere of an inert gas such as nitrogen and argon.
The reaction can be carried out in the presence or absence of a solvent. The solvent to be used in the above-mentioned reaction is not particularly limited as long as the solvent dissolves a starting compound to some extent and does not inhibit the reaction. Specific examples of the solvents include amides such as formamide, dimethylformamide, dimethylacetamide, hexamethylphosphoric triamide, and N-methyl pyrrolidone; aromatic hydrocarbons such as toluene, benzene, xylene, and mesitylene; ethers such as diethyl ether, diisopropyl ether, tetrahydrofuran, dioxane, dimethoxyethane, and diethylene glycol dimethyl ether; alcohols such as methanol, ethanol, n-propanol, isopropanol, n-butanol, isobutanol, tert-butanol, isoamyl alcohol, diethylene glycol, glycerine, octanol, cyclohexanol, and methyl cellosolve; nitriles such as acetonitrile, and isobutyronitrile; sulfoxides such as dimethyl sulfoxide, and sulfolane; esters such as methyl acetate, ethyl acetate, propyl acetate, and diethyl carbonate; or water or mixture solvent thereof. Preferable examples include nitriles, and a more preferable example is acetonitrile.
The reaction temperature is not particularly limited, but the preferable reaction temperature ranges from -30°C to a refluxing temperature of the solvent, and more preferable reaction temperature ranges from 0°C to 30°C.
The reaction time is not particularly limited, but preferable reaction time ranges from 10 minutes to 48 hours, and more preferable time ranges from 30 minutes to 2 hours.

The above-mentioned reaction may be carried out in the presence of a base. The base is not particularly limited as long as, for example, it can obtain a target compound and does not produce non-separable byproducts. Specific examples of the base include inorganic bases such as tripotassium phosphate, trisodium phosphate, cesium carbonate, potassium carbonate, sodium carbonate, cesium hydrogencarbonate, potassium hydrogencarbonate, sodium hydrogencarbonate, sodium acetate, barium hydroxide, potassium hydroxide, potassium fluoride, and cesium fluoride; and organic bases such as metal alkoxides, for example, sodium ethoxide, and sodium-tert-butoxide, acetates or alkali metals, for example, sodium acetate and potassium acetate, or triethyl amine, and the like. Preferably, the base is organic bases, and more preferably, the base is triethylamine.
The base can be used in an amount of 0.5 to 5 molar equivalents with respect to 1 mole of the compound represented by the above-mentioned formula (II-a) (hereinafter, also referred to as a compound (II-a). The same is true to compounds represented by the other formula.), a compound (II), a compound (II-b) or a compound (II-c), or a salt thereof Preferably, the base is used in an amount of 1 to 3 molar equivalents. Note here that the amount of the base to be used can be appropriately increased or decreased in the range of 0.5 to 2 equivalents depending upon salts as raw materials to be used.

Furthermore, the above-mentioned reaction may be carried out in the presence of a condensation agent. Examples of the condensation agent include isobutyl chloroformate, dicyclohexylcarbodiimide (DCC), water soluble carbodiimide (WSC), 2-ethoxy-N-ethoxycarbonyl-1,2-dihydroquinoline, benzotriazole -1-yl-oxy-tris-(dimethylamino)phosphonium hexafluorophosphate (BOP), diethyl azodicarboxylate-triphenylphosphine, diethyl cyanophosphonate (DEPC), diphenylphosphoryl azide (DPPA), Bromotripyrrolidinophosphonium hexafluorophosphate (PyBrop [trademark]), bis(2-oxo-3-oxazolidinyl) phosphinic chloride (BOPCl), benzotriazol-1-yl-oxy-tris-pyrrolidino-phosphonium hexafluorophosphate (PyBOP), 2-(1-H-benzotriazol-l-yl)-1,1,3,3,-tetramethyluronium hexafluorophosphate (HBTU), ethyl chloroformate, chlorodimethoxy-triazine (CDMT), and the like. A preferable condensation agent is isobutyl chloroformate.
The condensation agent may be used in an amount of 0.5 to 5 molar equivalents with respect to one molar equivalent of the compound (II-a), the compound (II), compound (II-b), or the compound (II-c). A preferable amount is 1 to 3 molar equivalents.
A dehydrating agent such as a molecular sieve may be added if necessary.

All of the compound (II-a), the compound (II), the compound (II-b), and the compound (II-c) are new compounds and can be synthesized according to Examples below or processes similar to Examples below.
Furthermore, the compound (II-a), the compound (II), the compound (II-b) and the compound (II-c) as well as the compound (I-a), the compound (I), the compound (I-b) and the compound (I-c) may be a salt. Specific examples of the salt include hydrohalides (for example, hydrofluoride, hydrochloride, hydrobromide, hydroiodide, and the like); inorganic acid salts (for example, sulfate, nitrate, perchlorate, phosphate, carbonate, bicarbonate, and the like), organic carboxylates (for example, acetate, oxalate, maleate, tartrate, fumarate, citrate, and the like); organic sulfonates (for example, methanesulfonate, trifluoromethanesulfonate, ethanesulfonate, benzene sulfonate, toluene sulfonate, camphorsulfonate, and the like), amino acid salts (for example, aspartate, glutamate, and the like), quaternary ammonium salt; alkali metal salts (for example, sodium salt, potassium salt, and the like), alkali earth metal salts (for example, magnesium salt, calcium salt, and the like), and the like. Preferable examples include hydrohalides, inorganic acid salts, and organic sulfonates, and more preferable examples include hydrochloride and hydrobromide.
Furthermore, the compound (II-a), the compound (II), the compound (II-b) and the compound (II-c) as well as the compound (I-a), the compound (I), the compound (I-b) and the compound (I-c) may include isomers such as an optical isomers and a stereoisomer, which are based on an asymmetric carbon, and isomer mixtures, which may include any one of the isomers or isomer mixtures.

Hereinafter, Examples of the present invention are described in more detail. However, the present invention is not necessarily limited to these examples. In the specification, room temperature represents temperatures in the range from 20°C to 30°C, and preferably about 254°C.

Furthermore, HPLC conditions are as follows. HPLC conditions:
Analysis Example 1: Analysis Example 1 was used in analysis in each step of Example 1.
Column: L-column (Trade mark) (150 × 4.6 mm LD., ODS 5 µm, Chemicals Evaluation and Research Institute)
Mobile phase:
Solution A: H₂O/MeCN/70%HClO₄ = 990/10/1 (v/v/v)
Solution B: H₂O/MeCN/70%HClO₄ = 100/900/1 (v/v/v)
Gradient (time (min)/Bconc. (%)):
0.01/40 → 5/40 → 18/100 → 25/100 → 25.01/40 → 30/stop Flow rate (mL/min): 1.0 mL/min
Oven temp: 354°C
Retention time of compound (Ib) : Rt = 7.4 min, raw material (IIb) : Rt = 2.9 min.

Analysis Example 2: Analysis Example 2 was used in analysis in each step of Example 2.
Column: L-column (Trade Mark) (150 × 4.6 mm I.D., ODS 5 µm, Chemicals Evaluation and Research Institute)
Mobile phase:
Solution A: H₂O/MeCN/70%HClO₄ = 990/100/1 (v/v/v)
Solution B: H₂O/MeCN/70%HClO₄ = 100/9001(v/v/v)
Gradient (time (min)/Bconc. (%)):
0/40 → 20/40 → 40/100 → 45/100 → 45.01/40 → 60/stomp Flow rate (mL/min): 1.0 mL/min
Oven temp: 40°C
Retention time of compound (Ic): Rt = 5.1 ± 0.5 min, raw material (IIc): Rt = 2.3 ± 0.5 min.

Note here that abbreviations shown in the structural formulae in Examples below are as follows.
Boc: tert-butoxycarbonyl group
Me: methyl group
Bn: benzyl group
Ph: phenyl group

### Example 1

### 1) Synthesis of methyl (2R)-2-[(tert-butoxycarbonyl)amino]hexanedioiate

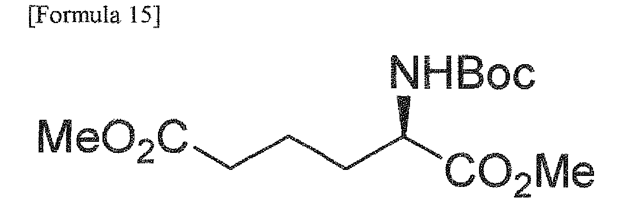

(2R)-2-Aminohexanedioic acid (100 g, 0.62 mol) was dissolved in methanol (1000 mL) with stirring under a stream of nitrogen, and the reaction solution was cooled to - 20°C. After thionyl chloride (100 mL, 1.37 mol) was added dropwise to the reaction solution, the reaction solution was stirred at 20°C for 18 hours. After the reaction solution was cooled so that the inner temperature was 9°C, an aqueous solution of potassium carbonate (36.4%, 430 mL) was added dropwise to the reaction solution. Then, di-tert-butyl-dicarbonate (176.4 g, 0.81 mol) was added thereto, and the solution was washed with methanol (20 mL). An aqueous solution of potassium carbonate (36.4%, 270 mL) was further added dropwise to the reaction solution, and the reaction solution was stirred at 20°C for 3.5 hours. Water (700 mL) was added to the reaction solution, followed by extraction with tert-butyl methyl ether (1000 mL). The organic layer was washed with 10% brine (300 mL). The solvent was removed by evaporation under reduced pressure, and azeotropic concentration with toluene was carried out under reduced pressure to give a crude product of the title compound (191.3 g). Yield: 94.5% (HPLC quantification).
¹H-NMR (CDCl₃) δ (ppm): 1.45 (9H, s), 1.60-1.75 (3H, in), 1.80-1.90 (1H, m), 2.30-2.40 (2H, m), 3.67 (3H, s), 3.75 (3H, s), 4.30-4.35 (1H, br-m), 5.00-5.10 (1H, br-d).

### 2) Synthesis of tert-butyl [(1R)-5-hydroxy-1-(hydroxymethyl)pentyl]carbamate

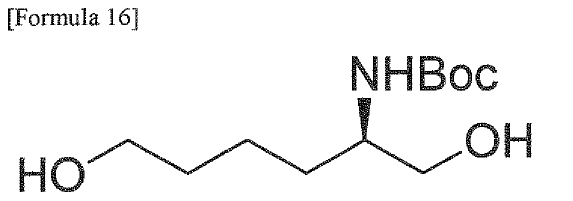

ARed-Al (Trade Mark) (Aldrich) toluene solution (65%, 806 g, 2.59 mol) was dissolved in tetrahydrofuran (1050 mL) under a stream of nitrogen. The reaction solution was cooled with stirring so that the inner temperature was -15.8°C. A solution of methyl (2R)-2-[(tert-butoxycarbonyl)amino]hexanedioiate in tetrahydrofuran (417 g, content: 36.0%, 0.518 mol) was added dropwise to the reaction solution (inner temperature: not higher than -5.7°C), and the reaction solution was stirred at 25°C for 18 hours. The reaction solution was fed to a 5 N aqueous solution of sodium hydroxide (2074 mL) that had been cooled to 8.4°C with stirring. After the reaction solution was rinsed with THF (66.7 g), tert-butyl methyl ether (1119 g) was put thereto. The aqueous layer was separated at 25°C, and then the organic layer was washed sequentially with a 20% ammonium chloride solution (750 mL) and a 20% brine (750 mL). The solvent was removed by evaporation under reduced pressure, and azeotropic concentration with toluene was carried out under reduced pressure to give a crude product of the title compound (116.1 g, content: 93.3%). Yield: 90.1%.
¹H-NMR (CDCl₃) δ (ppm): 1.45 (9H, s), 1.20-1.74 (6H, m), 2.40-2.60 (1H, br-s), 3.46-3.60 (2H, br-m), 3.60-3.74 (4H, br-m), 4.60-4.78 (1H, br-m).

### 3) Synthesis of tert-butyl (4R)-4-(4-hydroxybutyl)-2,2-dimethyl-1,3-oxazoline-3-carboxylate

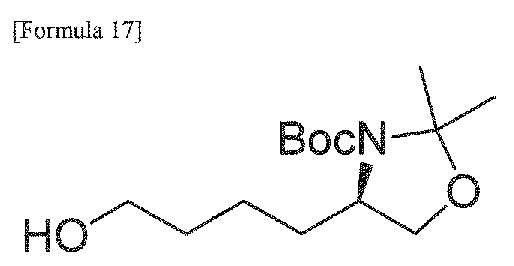

To a solution of a crude product of tert-butyl [(1R)-5-hydroxy-1-(hydroxymethyl)pentyl]carbamate (280 g, content: 83.3%, 1.20 mol) in acetone (840 mL), 2,2-dimethoxy propane (738 mL, 6.00 mol) and (1S)-10-camphorsulfonate (28 g, 0.12 mol) were added at 22°C under nitrogen atmosphere. The solution was stirred at the same temperature for 19 hours. After the solution was cooled so that the inner temperature was -14°C, water (840 mL) was added dropwise, and the solution was stirred for 40 minutes. To the solution, 5% sodium bicarbonate water (840 mL) was added, followed by extraction with tert-butyl methyl ether (3200 mL). The aqueous layer was further extracted with tert-butyl methyl ether (1400 mL). The combined organic layers were washed with 20% brine (840 mL). Then, the solvent was removed by evaporation under reduced pressure to give a crude product of the title compound (297.2 g, content: 85.8%). Yield: 93.0%.
¹H-NMR (CDCl₃) δ (ppm): 1.34-1.42 (2H, m), 1.45 (9H, s), 1.58 (6H, brs), 1.42-1.75 (4H, m), 1.75-1.80 (1H, m), 3.70-3.95 (1H, br), 3.64-3.70 (2H, brd-like), 3.74 (1H, d-like, Jav. = 7.6 Hz), 3.93 (1H, dd, J = 4.6, 8.6 Hz).

### 4) Synthesis of tert-butyl (4R)-4-[4-(benzyloxy)butyl]-2,2-dtmethyl-1,3-oxazoline-3-carboxylate

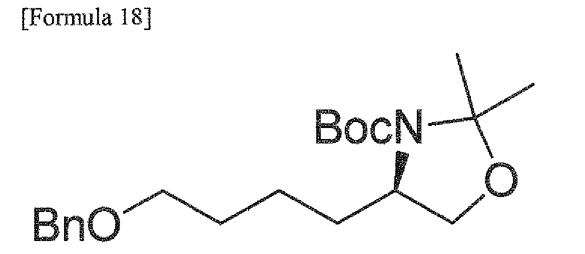

After a suspension of potassium tert-butoxide (3.5 g, 31.0 mmol) in tetrahydrofuran (20 mL) was cooled to -15°C under nitrogen atmosphere, a solution of a crude product of tort-butyl (4R)-4-(4-hydroxybutyl)-2,2-dimethyl-1,3-oxazoline-3-carboxylate (6.00 g, content: 94.2%) in tetrahydrofuran (8 mL) was added dropwise thereto. The solution was stirred at the same temperature for one hour. Benzyl bromide (3.20 mL) was added dropwise to the solution, and the solution was stirred at -20°C for 17 hours. After a 40% aqueous solution of dimethylamine (2.33 g) was added to the reaction solution, the reaction solution was stirred for 30 minutes while it was heated so that the inner temperature was 50°C. The reaction solution was cooled so that the inner temperature was 9°C, and then water (28 mL) and n-heptane (28 mL) were added, followed by extraction. The organic layer was washed sequentially with 10% aqueous solution of potassium hydrogen sulfate (28 g), 5% sodium bicarbonate water (28 g), and water (17 mL). Then, the solvent was removed by evaporation under reduced pressure to give a crude product of the title compound (7.93 g, content: 98.7%). Yield: >99%. ¹H-NMR (CDCl₃) δ (ppm): 1.46 (9H, s), 1.48 (3H, s), 1.61 (3H, s), 1.20-1.90 (6H, m), 3.47 (2H, t, J = 6.6 Hz), 3.70-3.95 (1H, br-m), 3.73 (1H, d-like, Jav. = 7.6 Hz), 3.91 (1H, dd, J = 5.2, 8.4 Hz), 4.50 (2H, br-s), 7.20-7.40 (5H, m).

### 5) Synthesis of tert-butyl [(1R)-5-(benzyloxy)-1-(hydroxymethyl)pentyl]carbamate

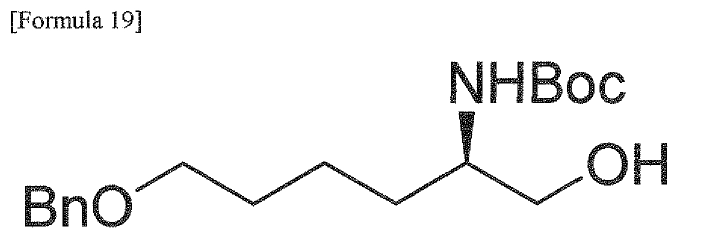

Tert-butyl (4R)-4-[4-(benzyloxy)butyl]-2,2-dimethyl-1,3-oxazoline-3-carboxylate (106.6 g, 293 mmol) was dissolved in methanol (533 mL) with stirring under a stream of nitrogen, and 5 N aqueous hydrochloric acid (106.6 mL) was added dropwise at the inner temperature of 7.5°C. The reaction solution was warmed to room temperature, and stirred at the same temperature for 2 hours. To the reaction solution, 14% brine (533 mL) was added, followed by extraction with toluene (1066 mL). The organic layer was washed sequentially with 5% sodium bicarbonate water (533 mL) and 10% brine. Then, the solvent was removed by evaporation under reduced pressure to give a crude product of the title compound (99.4 g, content: 89.3%). Yield: 93.7%.
¹H-NRR (CDCl₃) δ (ppm): 1.45 (9H, s), 1.20-2.90 (6H, m), 2.30-2.50 (1H, br-s), 3.48 (2H, t, J = 6.4 Hz), 3.50-3.80 (3H, m), 4.50 (2H, br-s), 4.62 (1H, br-s), 7.20-7.40 (5H, m).

### 6) Synthesis of tert-butyl ({(2R)-6-(benzyloxy)-2-[(tert-butoxycarbonyl)amino]hexyl}loxy)ethanoate

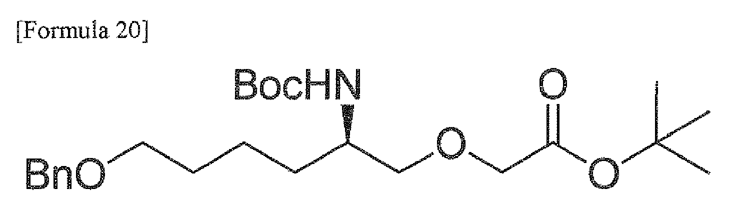

A crude product of tert-butyl [(1R)-5-(benzyloxy)-1-(hydroxymethyl)pentyl]carbamate (98 g, content: 89.3%, 271 mmol) was dissolved in toluene (788 mL) with stirring under a stream of nitrogen. To this solution, N,N,N,N-tetra(n-butyl)ammonium hydrogen sulfate (18.4 g, 54.2 mmol) was added, and the solution was cooled so that the inner temperature was 7.8°C. After 25% aqueous solution of sodium hydroxide (315 mL) was added to the reaction solution, tert-butyl 2-bromoethanoate (120 mL, 818 mmol) was added. The solution was stirred at the same temperature for 21 hours. After the reaction solution was warmed so that the inner temperature was 20°C, a 50% aqueous solution of dimethylamine (142.6 mL) was added thereto and the reaction solution was stirred at the same temperature for one hour. To the reaction solution, a 10% aqueous solution of potassium hydrogen sulfate (1401 mL) was added and mixed. Then, the aqueous layer was separated, and the organic layer was washed sequentially with 5% sodium bicarbonate water (438 mL), and 5% brine (438 mL). Then, the solvent was removed by evaporation under reduced pressure to give a crude product of the title compound (139.8 g, content: 76.8%). Yield: 90.6%.
¹H-NMR (CDCl₃) δ (ppm): 1.40-1.70 (6H, m), 1.44 (9H, s), 1.48 (9H, s), 3.47 (2H, t, J = 6.4 Hz), 3.40-3.50 (1H, m), 3.56 (1H, dd, J = 3.8, 9.4 Hz), 3.60-3.75 (1H, br-s), 3.95 (2H, s), 4.50 (2H, s), 4.93 (1H, br-d, Jav. = 7.2 Hz), 7.20-7.40 (5H, m).

### 7) Synthesis of ({(2R)-6-(benzyloxy)-2-[(tert-butoxycarbonyl)amino]hexyl}oxy)ehtanoate

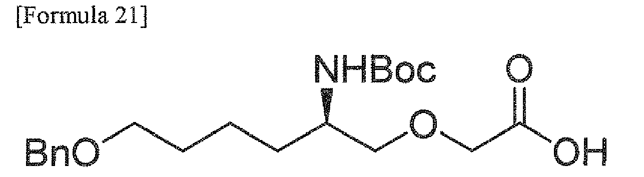

A crude product of tert-butyl ({(2R)-6-(benxyloxy)-2-[(tert-butoxycarbonyl)amino]hexyl}oxy)ethanoate (363.0 g, content: 79.9%, 662.7 mmol) was dissolved in tetrahydrofuran (870 mL) under a stream of nitrogen. Methanol (145 mL) was added thereto at room temperature with stirring. After 5 N aqueous solution of sodium hydroxide (290 mL) was put into the reaction solution at the same temperature, the reaction solution was stirred for one hour after the inner temperature was conformed to be 45°C. The reaction solution was cooled so that the inner temperature was 20°C, n-heptane (1740 mL) and water (2900 mL) were added to the reaction solution, then the mixture was allowed to stand, and the organic layer was separated. The aqueous layer was cooled so that the inner temperature was 11°C, 5 N aqueous hydrochloric acid (377 mL) was added and neutralized, followed by extraction with ethyl acetate (2900 mL). The organic layer was washed with 10% brine (870 mL) twice. Then, the solvent was removed by evaporation under reduced pressure to give a crude product of the title compound (289.2 g, 89.6%). Yield: 98.9%.
¹H-NMR (CDCl₃) δ (ppm): 1.40-1.70 (4H, m), 1.44 (9H, s), 1.56-1.70 (2H, m), 3.47 (2H, t, J = 6.4 Hz), 3.53 (2H, d, J = 4.8 Hz), 3.70-3.85 (1H, br-s), 4.10 (2H, s), 4.50 (2H, s), 4.67 (1H, br-s), 7.25-7.35 (5H, m).

### 8) Synthesis of ({(2R)-6-(benzyloxy)-2-[(tert-butoxycarbonyl)amino]hexyl}oxy)ethanoate dicyclohexylamine (1:1) (seed crystal)

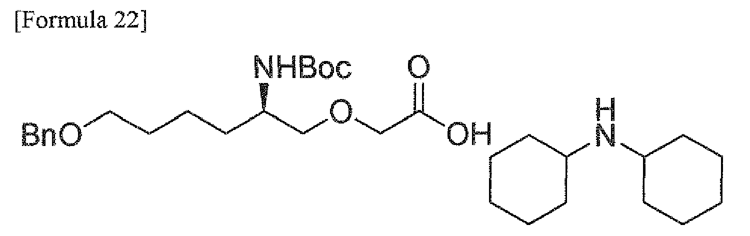

A crude product of ({(2R)-6-(benzyloxy)-2-[(tert-butoxycarbonyl)amino]hexyl}oxy)ethanoate (555 mg, content: 92.0%, 1.34 mmol) was dissolved in ethyl acetate (2.5 mL) and n-heptane (5.0 mL) at room temperature under nitrogen atmosphere. After dicyclohexylamine (265 mg, 1.46 mmol) was added to the reaction solution, the reaction solution was stirred at the same temperature for 44 hours. Precipitated crystals were filtered out, washed with n-heptane (1.67 mL) 3 times, and dried under reduced pressure to give the title compound (1.61 g). Yield: >99%.

### 9) Synthesis of ({(2R)-6-(benzyloxy)-2-[(tert-butoxycarbonyl)amino]hexyl}oxy)ethanoate dicyclohexylamine (1:1)

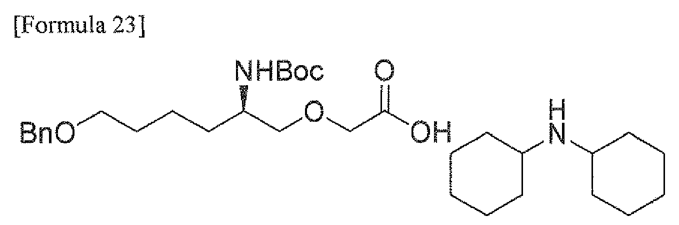

A crude product of ({(2R)-6-(benzyloxy)-2-[(tert-butoxycarbonyl)amino]hexyl}oxy)ethanoate (267.9 g, content: 89.6%, 629 mmol) was dissolved in ethyl acetate (2400 mL) under a stream of nitrogen, and n-heptane (1200 mL) was further added thereto. Then, the solution was stirred at 23°C. Dicyclohexylamine (138 mL, 692 mmol) was added to the solution, then the seed crystal (240 mg) obtained in Example 8 was added thereto, and the solution was stirred at the same temperature for 21 hours. Precipitated crystals were filtered out and washed with a 1:1 fixture solution (480 mL) of ethyl acetate : n-heptane, and dried by ventilation to give the title compound (369 g). Yield: >99%.
¹H-NMR (d₆-DMSO) δ (ppm): 1.00-1.38 (6H, m), 1.35 (9H, s), 1.40-1.60 (5H, m), 1.68 (4H, br-d, J = 12.0 Hz), 1.86 (4H, br-d, J = 13.2 Hz), 2.72-2.88 (2H, br-s), 3.10-3.50 (13H, m), 3.38 (2H, t, J = 6.4 Hz), 3.67 (2H, br-s), 4,12 (2H, s), 7.10-7.35 (5H, m).

### 10) Synthesis of (5R)-5-[4-(benzyloxy)butyl]morpholin-3-one

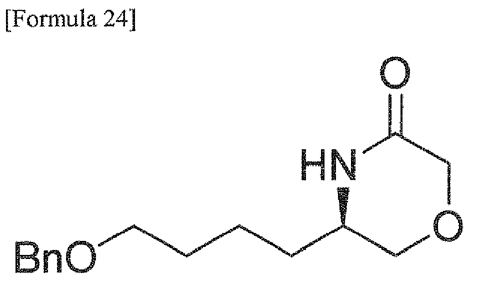

To an ethyl acetate (2170 mL)/acetone (930 mL) suspension of ({(2R)-6-(benzyloxy)-2-[(tert-butoxycarbonyl)amino]hexyl}oxy)ethanoate dicyclohexylamine (1:1) (310.0 g, 550.8 mmol), 5% sulfuric acid solution (3100 g) was added dropwise with stirring at the inner temperature of 7.5°C under a stream of nitrogen. After stirring for 17 minutes, the organic layer was separated, and concentrated under reduced pressure. Methanol (930 mL) was added to the residue, which was concentrated under reduced pressure. Then, the resultant crude product (239.8 g) was dissolved in 930 mL of methanol. The solution was cooled at 8°C with stirring and, and concentrated sulfuric acid (64.6 mL) was added dropwise to the solution. The mixture was stirred for 2.5 hours while it was warmed in a water bath of 45°C. The mixture was cooled so that the inner temperature was 9.74°C, and then a 28% sodium methoxoide methane solution (360 mL) was added dropwise thereto, and the solution was stirred for 30 minutes. Furthermore, 28% sodium methoxoide methanol solution (36 mL) was added, and the solution was stirred for one hour. Then, 5 N aqueous hydrochloric acid (60 mL) was added. After ethyl acetate (3100 mL) and water (2170 mL) were added to the solution, the solution was separated. Then, the organic layer was washed with 10% brine (930 mL), and the solvent was removed by evaporation under reduced pressure. Furthermore, tert-butyl methyl ether (620 mL) was added, and azeotropic concentration was carried out to give a crude product of the title compound (118.2 g, content: 92.4%). Yield: 75.3%.
¹H-NMR (CDCl₃) δ (ppm): 1.40-1.85 (6H, m), 3.47 (1H, d, J = 5.6 Hz), 3.47 (2H, t, J = 6.4 Hz), 3.50-3.55 (1H, m), 3.87 (1H, dd, J = 3.2, 11.2 Hz), 4.10 (1H, d, J = 16.8 Hz), 4.18 (1H, d, J = 16.8 Hz), 4.50 (2H, s), 6.50 (1H, br-s), 7.25-7.40 (5H, m).

### 11) Synthesis of tert-butyl (3R)-3-[4-(benzyloxy)butyl]-5-oxomorpholine-4-carboxylate

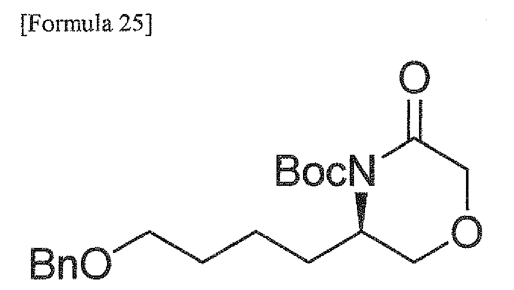

(5R)-5-[4-(Benzyloxy)butyl]morpholin-3-one (108.0 g, 410.1 mmol) was dissolved in tert-butyl methyl ether (1000 mL) under a stream of nitrogen, and the solution was stirred at the inner temperature of 22°C. After di-tert-butyl-dicarbonate (134.3 g, 615.2 mmol) was added dropwise to the reaction solution, 4-N,N-dimethylamino pyridine (5.01 g, 41.0 mmol) was added thereto at the same temperature, and the solution was stirred for 16 hours. The mixture solution was cooled to 7.74°C, imidazole (28 g, 410.1 mmol) was added thereto, and the mixture solution was stirred for 85 minutes. After the reaction solution was diluted with toluene (1000 mL), the organic layer was washed sequentially with 1% aqueous hydrochloric acid (1080 mL), 5% sodium bicarbonate water (540 mL), and 5% brine (540 mL). The solvent was removed by evaporation under reduced pressure to give a crude product of the title compound (187.8 g, content: 83.3%). Yield: >99%. ¹H-NMR (CDCl₃) δ (ppm): 1.35-1.60 (2H, m), 1.45 (9H, s), 1.60-1.70 (3H, m), 1.80-1.85 (1H, m), 3.48 (2H, t, J = 6.4 Hz), 3.72 (1H, d, J = 12.4 Hz), 3.95 (1H, d, J = 12.4 Hz), 3.98-4.30 (1H, m), 4.15 (1H, d, J = 17.2 Hz), 4.27 (1H, d, J = 17.2 Hz), 4.50 (2H, s), 7.20-7.40 (5H, m).

### 12) Synthesis of tert-butyl (3R)-3-[4(benzyloxy)butyl]-5-[(diphenoxyphosphoryl)oxy]-2,3-dihydro-4H-1,4-oxazine-4-carboxylate

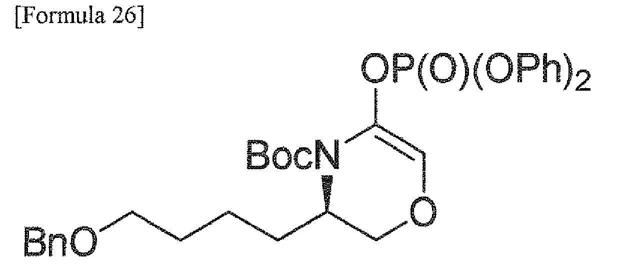

Tert-butyl (3R)-3-[4-(benzyloxy)buty]-5-oxomorpholine-4-carboxylate (63.0 g, content: 79.4%) was dissolved in tetrahydrofuran (230 mL) and toluene (150 mL) under a stream of nitrogen. Then, the solution was cooled to -25°C. Diphenyl chlorophosphate (31.4 mL) was added to the solution, the solution was washed with tetrahydrofuran (12.5 mL). Lithium hexamethyldisilazide tetrahydrofuran solution (1.0 M: 165.1 mL, 165.1 mmol) was added dropwise to the mixture solution at the same temperature. The solution was stirred for 18 hours. To the solution, a 10% aqueous solution of ammonium chloride (500 g) and toluene (250 mL) were added, followed by extraction. Then, the organic layer was washed sequentially with a 10% aqueous solution of ammonium chloride (500 g), a 5% sodium bicarbonate water (500 g), 5% brine (250 mL), and water (250 mL). The solvent was removed by evaporation under reduced pressure. To the residue, toluene (150 mL) was added, and azeotropic concentration was carried out under reduced pressure to give a crude product of the title compound (100.3 g, content: 89.3%). Yield: >99%. ¹H-NN4R (CDCl₃) δ (ppm): 1.35-1.75 (6H, m), 1.45 (9H, s), 3.35 (2H, t, J = 6.4 Hz), 3.69 (1H, dd, J = 2.8, 11.2 Hz), 4.02 (1H, d, J = 11.2 Hz), 4.30-4.35 (1H, m), 4.43 (2H, s), 6.30 (1H, d, J = 3.6 Hz), 7.15-7.40 (15H, m).

### 13) Synthesis of tert-butyl (3R)-3-[4-(benzyloxy)butyl]-5-(3,4,5-trifluoropheny)-2,3-dihydro-4H-1,4-oxazine-4-carboxylate

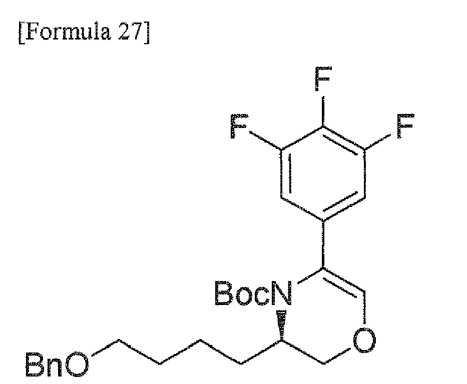

Tert-butyl (3R)-3-[4-(benzyloxy)butyl]-5-[(diphenoxyphosphoryl)oxy]-2,3-dihydro-4H-1,4-oxazine-4-carboxylate (17.4 g, 29.2 mmol) was dissolved in N,N-dirnethylformamide (87.0 mL) and toluene (17.4mL). To the solution, water (1.05 mL, 58.4 mmol) and 3,4,5-trifluorophenylboronic acid (7.71 g, 43.8 mmol) were added and subjected to nitrogen-substitution. Then, bis (triphenylphosphine)palladium dichloride (2.05 g, 2.92 mmol) and cesium carbonate (19.0 g, 58.4 mmol) were added and subjected to nitrogen-substitution again. The reaction solution was stirred at the inner temperature of 85°C to 106°C for 2.5 hours, and cooled so that the inner temperature was 50°C. To the reaction solution, 50% aqueous solution of methanol (174 g) was added, and n-heptane (139 mL) was added at the inner temperature of 35°C. The mixture was filtered through celite, and rinsed with n-heptatte (35 mL) and separated. The organic layer was washed with a 50% aqueous solution of methanol (174 g) twice and with 5% brine (87 g) and water (87 g), sequentially. The solvent was removed by evaporation under reduced pressure, azeotropic concentration with n-heptane (52 mL) was carried out under reduced pressure, and substitution concentration with methanol (87 mL) was carried out under reduced pressure to give a crude product of the title compound (11.8 g). Yield: 84.8%.
¹H-NMR (CDCl₃) δ (ppm): 1.20 (9H, s), 1.40-1.50 (1H, m), 1.35-1.50 (5H, m), 3.51 (2H, t, J = 6.4 Hz), 3.93 (1H, dd, J = 2.8, 10.8 Hz), 4.15 (1H, dd, J = 1.2, 10.8 Hz), 4.40-4.45 (1H, m), 4.51 (2H, s), 6.18 (1H, s), 6.80 (2H, dd-like, J = 6.4, 8.8 Hz), 7.15-7.40 (5H, m).

### 14) Synthesis of tert-butyl (3R,5S)-3-(4-hydroxybutyl)-5-(3,4,5-trifluorophenl)morpholine-4-carboxylate

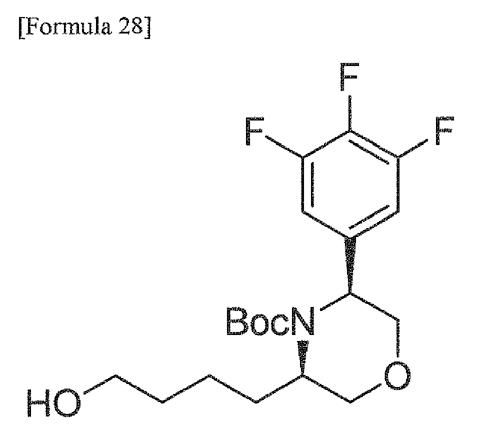

Tert-butyl (3R)-3-[4-(benzyloxy)butyl]-5-(3,4,5-trifluorophenyl)-2,3-dihydro-4H-1,4-oxazine-4-carboxylate (4.83 g, 10.1 mmol) was dissolved in methanol (24.2 mL). To the solution, 20% palladium hydroxide-C (50% water content, 2.42 g) was added. The solution was stirred for 19 hours under hydrogen pressure (2 MPa) in a state in which the jacket temperature was set to 704°C. After the mixture was filtered through celite, the filter cake was washed with methanol (10 mL). The filtrate was concentrated under reduced pressure, and substitution concentrated with acetonitrile (24 mL) under reduced pressure to give a crude product of the title compound (3.63 g, content: 89.5%) as a brown oil. Yield: 88.2%. ¹H-NMR (CDCl₃) δ (ppm): 0.98-1.10 (1H, m), 1.20-1.80 (6H, m), 1.52 (9H, s), 3.45-3.55 (2H, m), 3.67 (1H, dd, J = 4.0, 11.6 Hz), 3.78 (1H, dd, J = 4.0, 12.4 Hz), 3.84 (1H, d, J = 11.6 Hz), 3.90-3.98 (1H, m), 4.42 (1H, d, J = 12.4 Hz), 5.09 (1H, d, J = 3.6 Hz), 7.32 (2H, dd-like, J = 6.8, 8.8 Hz).

### 15) Synthesis of 4-[(3R,5S)-4-(tert-butoxycarbonyl)-5-(3,4,5-trifluorophenyl)morpholin-3-yl]butanoic acid

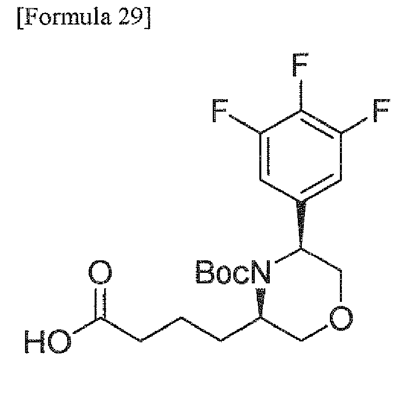

A crude product of tert-butyl (3R,5S)-3-(4-hydroxybutyl)-5-(3,4,5-trifluorophenyl)morpholine-4-carboxylate (2.15 g, content: 86.4%, 5.51 mmol) was dissolved in acetonitrile (10.7 mL) and water (14.7 mL) with stirring at room temperature under nitrogen atmosphere. The solution was cooled to 8°C. After sodium hydrogen carbonate (463 mg) and 2,2,6,6-tetramethylpiperidine-1-oxyl (TEMPO) (86.1 mg) were added to the reaction solution, an aqueous solution of sodium hypochlorite (available chlorine content: 11.9%, 8.59 mL) was added dropwise to the reaction solution at the same temperature in a state in which the inner temperature was not higher than 20°C. After stirring for one hour, to the reaction solution, toluene (10.7 mL) was added, and a 10% aqueous solution of sodium sulfite (10.7 mL) was further added. The reaction solution was warmed to room temperature and stirred for 30 minutes. Then, to the reaction solution, 5 N aqueous hydrochloric acid (3.22 mL) was added dropwise thereto, and the aqueous layer was separated. To the organic layer was added 1 N aqueous solution of sodium hydroxide (21.5 mL) with stirring to remove the organic layer, followed by extraction with ethyl acetate (21.5 mL). The organic layer was washed sequentially with 5% brine (10.7 mL) and water (10.7 mL). The solvent was removed by evaporation under reduced pressure to give a crude product of the title compound (2.5 g, content: 92.8%). Yield: >99%.
¹H-NMR (CDCl₃) δ (ppm): 1.00-1.40 (4H, m), 1.52 (9H, s), 2.05-2.20 (2H, m), 3.68 (1H, d, J = 12.0 Hz), 3.78 (1H, d, = 8.4 Hz), 3.84 (1H, d, J = 12.0 Hz), 3.90-3.95 (1H, m), 4.42 (1H, d, ,T - 12.4 Hz), 5.11 (1H, br-d), 7.33 (2H, dd-like, J = 6.8, 8.8 Hz).

### 16) synthesis of 4-[(3R,5S)-5-(3,4,5-trifluorophenyl)morpholin-3-yl]butanoic acid hydrochloride (seed crystal)

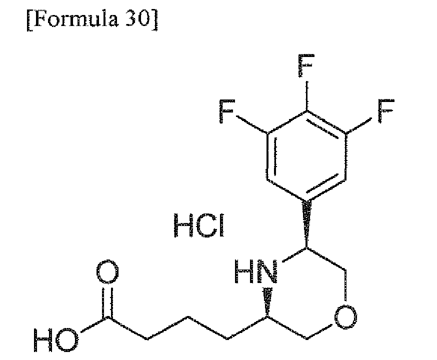

4-[(3R,5S)-4-(Tert-butoxycarbonyl)-5-(3,4,5-trifluorophenyl)morpholin-3-yl]butanoic acid (420 mg, 1.04 mmol) was dissolved in 1,2-dimethoxyethane (2.1 mL) under nitrogen atmosphere, and concentrated hydrochloric acid (0.186 mL, 2.09 mmol) was added thereto with stirring, which was heated at 50°C for 62.5 hours. The mixture was cooled to room temperature and stirred for 45 minutes. Then, the suspension was further cooled to 4°C and stirred for 2.3 hours. Crystals were filtered out, washed with 1,2-dimethoxyethane (1 mL) that had been cooled to 4°C, and dried under reduced pressure at 40°C to give the title compound (221 mg). Yield: 62.5%.
¹H-NMR (d₆-DMSO) δ (ppm): 1.28 (2H, m), 1.67-1.75 (2H, m), 2.24 (2H, t, J = 6.0 Hz), 3.34 (1H, m), 3.72 (1H, t-like, J = 11.6 Hz), 3.84 (1H, t-like, J = 11.6 Hz), 3.94 (1H, dd, J = 3.3, 12.1 Hz), 4.01 (1H, dd, J = 3.3, 12.1 Hz), 4.51 (1H, d, J = 6.4 Hz), 7.82 (2H, dd-like, J = 6.8, 8.8 Hz), 9.68 (1H, brs), 10.18 (1H, brs), 12.11 (1H, brs).

### 17) Synthesis of 4-[(3R,5S)-5-(3,4,5-trifluorophenyl)morpholin-3-yl]butanoic acid hydrochloride

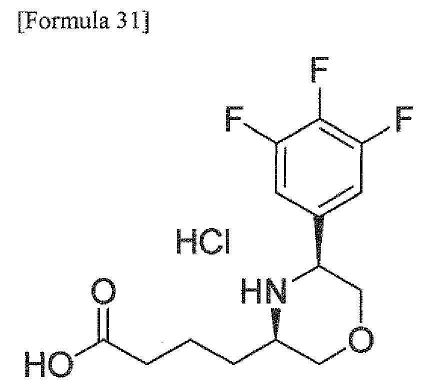

4-[(3R,5S)-4-(Tert-butoxycarbonyl)-5-(3,4,5-trifluorophenyl)morpholin-3-yl]butanoic acid (880 mg, 2.18 mmol) was dissolved in 1,2-dimethoxyethane (2.4 mL) under nitrogen atmosphere, and concentrated hydrochloric acid (0.354 mL, 3.98 mmol) was added thereto with stirring. The mixture was heated at 60°C. The mixture was cooled to room temperature. Then, the seed crystals (about 1 mg) of the title compound obtained in Example 16 were added to the mixture at the same temperature, and the mixture was stirred for 2 hours and 10 minutes. Ethyl acetate (8.0 mL) was added dropwise to the mixture over 15 minutes, and the suspension was cooled to 4°C and stirred for 11 hours. Precipitated crystals were filtered out, washed with ethyl acetate (that had been cooled to 4°C, 1.6 mL), and dried under reduced pressure at 40°C to give the title compound (741 mg). Yield: 55.7%.

### 18) Synthesis of (4S,9aR)-4-(3,4,5-trifluorophenyl)hexahydropyrido[2,1-c][1,4]oxazin-6(1H)-one

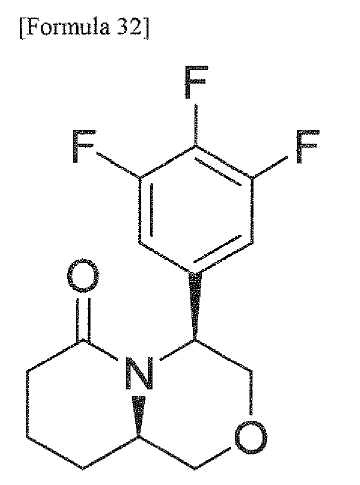

### i) Intramolecular condensation reaction

Isobutyl chloroformate (0.091 mL, 0.704 mmol) was added dropwise to a solution of 4-[(3R,5S)-5-(3,4,5-trifluorophenyl)morpholin-3-yl]butanoic acid hydrochloride (200 mg, 0.587 mmol) and triethylamine (0.204 mL, 1.47 mmol) in acetonitrile (2.0 mL) at 0°C under a stream of nitrogen. The mixture was warmed to room temperature and stirred for 2 hours. Tert-butyl methyl ether (2 mL) and water (1 mL) were added to the reaction mixture, and the organic layer was extracted. Then, the organic layer was washed sequentially with 1 N aqueous hydrochloric acid, 5% sodium bicarbonate water, 5% brine, and water, and dried over magnesium sulfate. The solvent was removed by evaporation under reduced pressure to give a crude product of the title compound. Yielded amount: 164 mg [as tert-butyl methyl ether solution (1.99 g), HPLC quantification]. Yield: 97.9%.

### ii) Crystallization purification

4-[(3R,5S)-5-(3,4,5-Trifluorophenyl)morpholin-3-yl]butanoic acid hydrochloride (358 mg, 1.05 mmol) was stirred at 50°C in a condition in which tert-butyl methyl ether (0.72 mL) was added to a crude product obtained by using tetrahydrofuran (3.58 mL) as a reaction solvent in the above-mentioned reaction conditions. After the seed crystal was added to the reaction solution, the reaction solution was cooled to room temperature. Then, n-heptane (2.15 mL) was further added to the reaction solution, and the reaction solution was stirred at the same temperature for one hour. The suspension was cooled to -30°C and stirred for 2 hours. Then, the crystal was washed with n-heptane, and dried under reduced pressure at 40°C to give the title compound (240 mg). Yield: 80.1%.
¹H-NMR (CDCl₃) δ (ppm): 1.50-1.60 (1H, m), 1.80-1.95 (2H, m), 1.95-2.05 (1H, m), 2.40-2.50 (2H, m), 3.56 (1H, t, J = 12.0 Hz), 3.62 (1H, dd, J = 6.4, 12.4 Hz), 3.80-3.90 (1H, m), 3.93 (1H, d, J = 10.8 Hz), 4.16 (1H, d, J = 8.4 Hz), 4.72 (1H, t-like, J = 6 Hz), 6.90 (2H, dd-like, J = 4.4, 12.8 Hz).

### Example 2

### 1) Synthesis of methyl (2S)-2-[(tert-butoxycarbonyl)amino]hexanedioiate

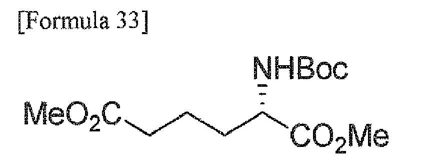

(2S)-2-Aminohexanedioic acid (500 g, 3.10 mol) was dissolved in methanol (5000 mL) with stirring under a stream of nitrogen, and the reaction solution was cooled to - 14°C. After thionyl chloride (812 g, 6.83 mol) was added dropwise to the reaction solution, the reaction solution was stirred at 20°C for 16 hours. The reaction solution was cooled so that the inner temperature was not higher than 9°C, and an aqueous solution of potassium carbonate (36.4%, 2830 g) was added dropwise the reaction solution. Then, di-tert-butyl-dicarbonate (880.0 g, 4.03 mol) was added thereto, the reaction solution was washed with methanol (100 mL). An aqueous solution of potassium carbonate (36.4%, 1860 mL) was added dropwise to the reaction solution, and the reaction solution was stirred at 25°C for 3 hours. To the reaction solution, water (3500 mL) was added, followed by extraction with tert-butyl methyl ether (5000 mL). The organic layer was washed with water (1500 mL). The solvent was removed by evaporation under reduced pressure, and azeotropic concentration with toluene was carried out under reduced pressure. The concentrated residue was dissolved in tetrahydrofuran (1500 mL) to give a solution of a crude product of the title compound in tetrahydrofuran (2184 g, content: 35.1%). Yield: 86.0% (HPLC quantification).

### 2) Synthesis of tert-but [(1S)-5-hydroxy-1-(hydroxymethyl)pentyl]carbamate

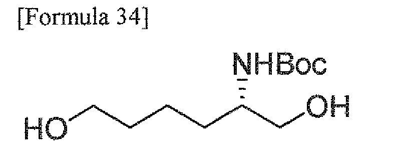

ARed-A1 (Trade Mark) (Aldrich) toluene solution (65%, 1979 g, 6.39 mol) was dissolved in tetrahydrofuran (2581 mL) under a stream of nitrogen, and the reaction solution was cooled with stirring so that the inner temperature was -13°C. To the reaction solution, a solution of methyl (2S)-2-[(tert-butoxycarbonyl)amino]hexanedioiate in tetrahydrofuran (1050 g, content: 35.1%, 1.27 mol) was added dropwise (inner temperature: not higher than -5.54°C), which was washed with tetrahydrofuran (57 mL). The reaction solution was stirred at 25°C for 18 hours and then cooled to 9.2°C. The reaction solution was fed to a 5 N aqueous solution of sodium hydroxide (4843 mL) that had been cooled to 10°C with stirring. Tert-butyl methyl ether (3688 mL) was put into the reaction solution. After the aqueous layer was separated, the organic layer was washed sequentially with a 20% aqueous solution of ammonium chloride (1844 mL) and 20% brine (1844 mL). The solvent was removed by evaporation under reduced pressure, and azeotropic concentration with toluene was carried out under reduced pressure. The residue was dissolved in acetone (738 mL) to give a solution of a crude product of the title compound in acetone (791.9 g, content: 33.2%). Yield: 88.3%.

### 3) Synthesis of tert-butyl (4S)-4-(4-hydroxybutyl)-2,2-dimethyl-1,3-oxazoline-3-carboxylate

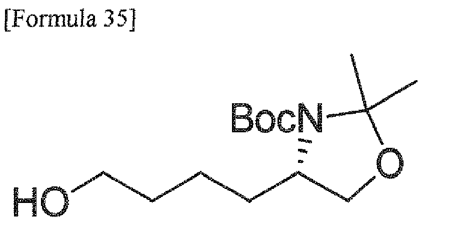

Acetone (193 mL) and 2,2-dimethoxy propane (1093.0 g, 10.5 mol) were added to a solution oftert-butyl [(1S)-5-hydroxy 1-(hydroxymethyl)pentyl]carbamate in acetone (a mixture of two lots: 737.3 g, content: 33.2%, 761.2 g, content: 32.2%; 2.10 mol) under nitrogen atmosphere. To the solution, (1S)-10-camphorsulfonate (48.7 g, 0.21 mol) was added with stirring, and the solution was stirred at 21°C for 19 hours. After the solution was cooled so that the inner temperature was -10°C, water (1469 mL) was added dropwise to the solution, and the solution was stirred for 50 minutes. To the solution was added 5% sodium bicarbonate water (1469 g), followed by extraction with tert-butyl methyl ether (4895 mL). The organic layer was washed with 5% brine (1469 g). Then, the solvent was removed by evaporation under reduced pressure, and azeotropic concentration with tert-butyl methyl ether was carried out under reduced pressure. The residue was diluted with tetrahydrofuran (490 mL) to give a solution of a crude product of the title compound in tetrahydrofuran (945.8 g, content: 51.9%). Yield: 85.5%.

### 4) Synthesis of tert-butyl (4S)-4-[4-(benzyloxy)butyl]-2,2-dimethyl-1,3-oxazoline-3-carboxylate

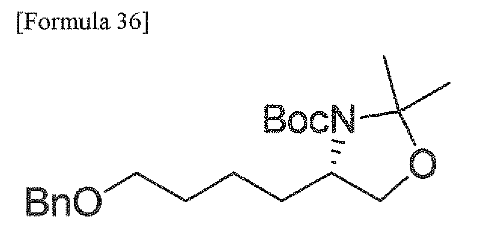

After a suspension of potassium tert-butoxide (302.1 g, 2.69 mol) in tetrahydrofuran (1717 mL) was cooled to -12°C under nitrogen atmosphere, a solution of a crude product of tert-butyl (4S)-4-(4-hydroxybutyl)-2,2-dimethyl-1,3-oxazoline-3-carboxylate in tetrahydrofuran (945.8 g, content: 51.9%, 1.79 mol) was added dropwise. The solution was stirred at the same temperature for one hour. To the solution were added dropwise benzyl bromide (399.1 g, 2.33 mol), followed by the rinse with tetrahydrofuran (24.6 mL). Then, the solution was stirred at -12°C for 22 hours. To the reaction solution, a 50% aqueous solution of dimethylamine (161.8 g, 1.79 mol) was added, and the reaction solution was then heated so that the inner temperature was 50°C and stirred for 60 minutes. While the reaction solution was cooled by circulating cool water (set to 8°C) to the jacket, and water (2429 g) was added to the reaction solution, followed by extraction with n-heptane (2453 mL). The organic layer was washed sequentially with 10% aqueous solution of potassium hydrogen sulfate (2453 g), 5% sodium bicarbonate water (2453 g), and water (1472 mL), and then the solvent was removed by evaporation under reduced pressure. The residue was dissolved in methanol (1472 mL) to give a solution of a crude product of the title compound in methanol (1796.2 g, content: 34.1%). Yield: 93.9%.

### 5) Synthesis of tert-butyl [(1S)-5-(benzyloxy)-1-(hydroxymethyl)pentyl]carbamate

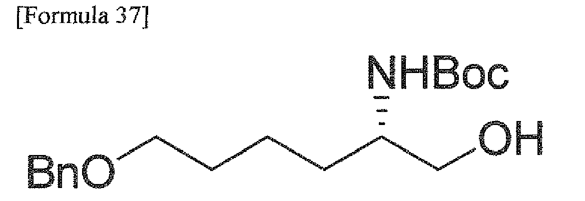

Methanol (1559 mL) was added to a solution of tert-butyl (4S)-4-[4-(benzyloxy)butyl]-2,2-dimethyl-1,3-oxazoline-3-carboxylate in methanol (1760.7 g, 1.65 mol) under a stream of nitrogen. To the solution, 5 N aqueous hydrochloric acid (600 mL) was added dropwise with stirring at the inner temperature of 11.4°C. The reaction solution was stirred at 9°C for 5 hours. To the reaction solution, 10% brine (3002 mL) was added, followed by extraction with toluene (6004 mL). The organic layer was washed sequentially with 5% sodium bicarbonate water (3002 mL) and 10% brine (3002 mL), and then the solvent was removed by evaporation under reduced pressure. The resultant residue was diluted with toluene to give a solution of a crude product of the title compound in toluene (2007.6 g, content: 24.5%). Yield: 92.1%.

### 6) Synthesis of tert-butyl ({(2S)-6-(benzyloxy)-2-[(tert-butoxycarbonyl)amino]hexyl}oxy)ethanoate

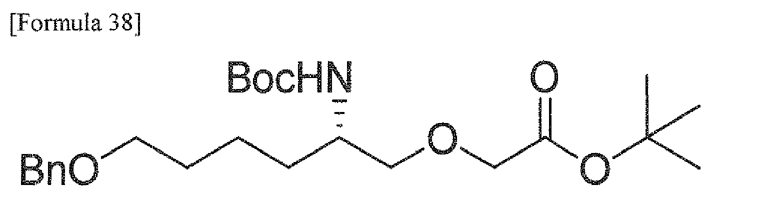

N,N,N,N-Tetra(n-butyl)ammonium hydrogen sulfate (90.8 g, 0.267 mol), a solution of a crude product tert-butyl [(1S)-5-(benzyloxy)-1-(hydroxymethyl)pentyl]carbamate in toluene (1984 g, content: 24.5%, 1.50 mol), and toluene (2097 mL) were sequentially put into a reactor vessel under a stream of nitrogen. The mixture solution was cooled so that the inner temperature was 11°C. To the reaction solution, 25% aqueous solution of sodium hydroxide (1557 mL) was added dropwise, then, tert-butyl 2-bromoethanoate (782.0 g, 4.01 mol) was added, and the reaction solution was stirred at 8 to 9°C for 22 hours. To the reaction solution, tert-butyl 2-bromoethanoate (10.6 g) was added, and the reaction solution was stirred for further 2 hours. Then, to the reaction solution, a 50% aqueous solution of dimethylamine (603.0 g) was added. The reaction solution was stirred at the same temperature for one hour. After the lower layer was taken out, a 10% aqueous solution of potassium hydrogen sulfate (6918 g) was added to the organic layer and mixed together, the aqueous layer was separated, and the organic layer was washed sequentially with 5% sodium bicarbonate water (2162 g) and 5% brine (2162 g). Then, the solvent was removed by evaporation under reduced pressure, and the residue was diluted with tetrahydrofuran (865 mL) to give a solution of a crude product of the title compound in tetrahydrofuran (1480.8 g, content: 44.6%). Yield; >99%.

### 7) Synthesis of ({(2S)-6-(benzyloxy)-2-[(tert-butoxycarbonyl)amino]hexyl}oxy)ethanoate

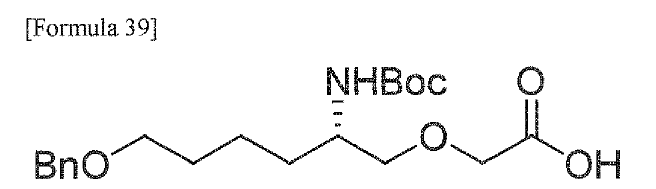

Tetrahydrofuran (1037 mL) and methanol (324 mL) were sequentially added to a solution of a crude product of tert-butyl ({(2S)-6-(benzyloxy)-2-[(tert-butoxycarbonyl)amino]hexyl}oxy)ethanoate in tetrahydrofuran (1451.2 g, content: 44.6%, 1.48 mol) under a stream of nitrogen. To the mixture solution, 5 N aqueous solution of sodium hydroxide (647 mL) was added at 15°C with stirring. After the jacket temperature was set to 60°C, the reaction solution was stirred at the temperature of the reaction solution of 50°C or above for 3 hours. After the reaction solution was cooled so that the inner temperature was 18°C, n-heptane (3883 mL) and water (6472 mL) were added and mixed together. The mixture was allowed to stand, and the organic layer was separated. The aqueous layer was cooled so that the inner temperature was 17°C, then neutralized by adding 5 N aqueous hydrochloric acid (841 mL), and extracted by adding ethyl acetate (6472 mL). The organic layer was washed with water (1942 mL) twice. Then, the solvent was removed by evaporation under reduced pressure, and azeotropic concentration with ethyl acetate was carried out under reduced pressure. The residue was diluted with ethyl acetate (994 mL) to give a solution of a crude product of the title compound in ethyl acetate (1824.8 g, content: 30.9%). Yield: 99.9%.

### 8) Synthesis of ({(2S)-6-(benzyloxy)-2-[(tert-butoxycarbonyl)amino]hexyl}oxy)ethanoate dicyclohexylamine (1:1)

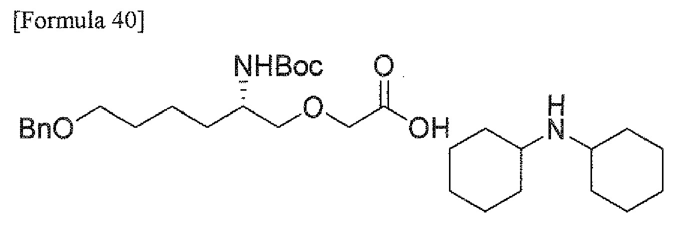

Ethyl acetate (4239 mL) and n-heptane (2818 mL) were added to a solution of a crude product ({(2S)-6-(benzyloxy)-2-[(tert-butoxycarbonyl)amino]hexyl}oxy)ethanoate in ethyl acetate (1824.8 g, content: 30.9%, 1.48 mol) under a stream of nitrogen, and the solution was stirred at 22°C. To the solution, dicyclohexylamine (323 mL), 1.63 mol) was added, and then the solution was vigorously stirred at 20°C to 25°C for 4 days. The jacket temperature was set to 50°C. The slurry was stirred at 40°C or above for 3 hours, and then gradually cooled to 204°C with stirring (cooling rate: 10°C/h). After stirring at 20°C for 17 hours, precipitated crystals were filtered out and the cake was washed with a 1:1 mixture solution of ethyl acetate : n-heptane (1128 mL), and dried by ventilation for one hour. The wet crystals were dried under reduced pressure at the outer temperature of 45°C to give the title compound (762.8 g). Yield: 91.7%.

### 9) Synthesis of (5S)-5-[4-(benzyloxy)butyl]morpholin-3-one

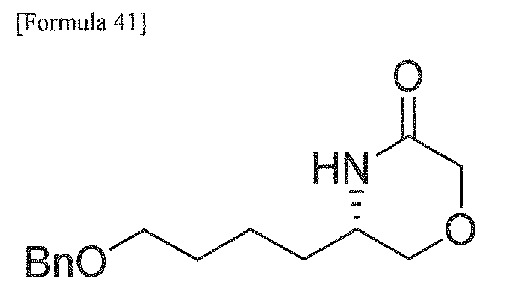

To an ethyl acetate (56 mL)/acetone (24 mL) suspension of ({(2S)-6-(benzyloxy)-2-[(tert-butoxycarbonyl)amino]hexyl}oxy)ethanoate dicyclohexylamine (1:1) (8.0 g, 14.2 mmol), a 5% sulfuric acid solution (80 g) was added dropwise with stirring at the inner temperature of 7.7°C under a stream of nitrogen. After the reaction solution was stirred at the same temperature for 15 minutes, methanol (4 mL) was added to the reaction solution, and the organic layer was separated and concentrated under reduced pressure, and then the residue was azeotropically concentrated with methanol under reduced pressure. Then, the resultant residue was diluted with methanol (24 mL). After the solution was cooled to 9°C with stirring, concentrated sulfuric acid (3.1 g) was added dropwise, and the mixture was stirred for 2 hours in a state in which the outer temperature was set to 50°C and the inner temperature was 40°C or above. The reaction solution was cooled so that the inner temperature was 11°C. Then, a 28% sodium methoxide methanol solution (12.1 g, 62.7 mmol) was added dropwise thereto, and the reaction solution was stirred for 11.5 hours. After tert-butyl methyl ether (64 mL) was added to the reaction solution, and the reaction solution was cooled to 4°C, concentrated sulfuric acid (0.5 mL) was added dropwise to the reaction solution with stirring so that the reaction solution was adjusted to pH 9. Water (24 mL) was added to the reaction solution. The reaction solution was warmed up to 23°C, subjected to suction filtration to remove insoluble matters, and rinsed with tert-butyl methyl ether (16 mL). The filtrate and the rinsing solution were concentrated under reduced pressure so that the amount of the solution became 28 g, tert-butyl methyl ether (80 mL) was added thereto, and the solution was separated. After the aqueous layer was extracted again with tert-butyl methyl ether (48 mL), the combined organic layers were washed with 10% brine (24.0 g), and the solvent was removed by evaporation under reduced pressure. Then, azeotropic concentration with tert-butyl methyl ether was carried out under reduced pressure to give a crude product of the title compound (3.70 g, content: 99.5%). Yield: 98.4%.

### 10) Synthesis of tert-butyl(3S)-3-[4-(benzyloxy)butyl]-5-oxomorpholine-4-carboxylate

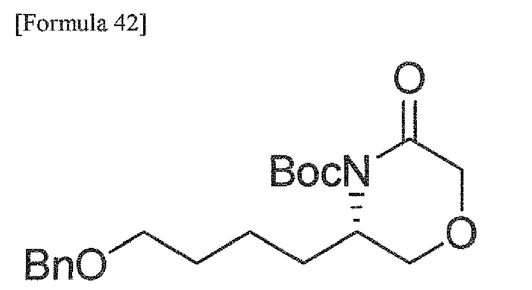

Tert-butyl methyl ether (632 mL) was additionally added to a solution of (5S)-5-[4-(benxyloxy)butyl]morpholin-3-one (223.7 g, 0.85 mol) in tert-butyl methyl ether (1535 mL) under a stream of nitrogen, and the solution was stirred at the inner temperature of 23°C. To the reaction solution, 4-N,N-dimethylamino pyridine (10.4 g, 0.085 mol) was added. Then, the reaction solution was cooled so that the inner temperature was 9°C and di-tert-butyl-dicarbonate (278.0 g, 1.27 mol) was added thereto, which was washed with tert-butyl methyl ether (70 mL). The reaction solution was stirred at 8 to 10°C for 16 hours. To the reaction solution, imidazole (57.8 g, 0.85 mol) was added, and the reaction solution was stirred for 30 minutes. After the reaction solution was diluted with toluene (2237 mL), the organic layer was washed twice with 1% aqueous hydrochloric acid (2237 mL) and then washed sequentially with 5% sodium bicarbonate water (1119 mL) and 5% brine (1119 mL). The solvent was removed by evaporation under reduced pressure, and azeotropic concentration with toluene was carried out under reduced pressure. The residue was diluted with toluene (224 mL) to give a solution of a crude product of the title compound in toluene (570.4 g, content: 49.7%). Yield: 91.7%.

### 11) Synthesis of tert-butyl (3S)-3-[4-(benzyloxy)butyl]-5-[(diphenoxyphosphoryl)oxy]-2,3-dihydro-4H-1,4-oxazine-4-carboxylate

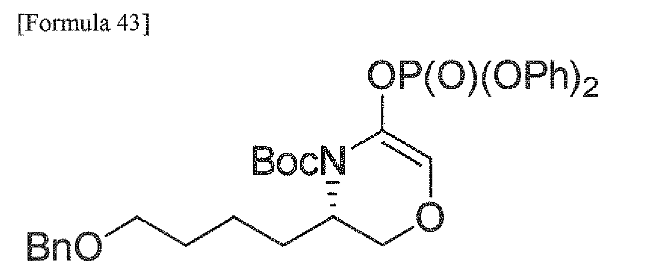

Toluene (515 mL), toluene (1205 mL) and diphenyl chlorophosphate (229.0 g, 0.85 mol) were sequentially added to a solution of a crude product of tert-butyl (3S)-3-[4-(benzyloxy)butyl]-5-oxomorpholine-4-carboxylate in toluene (567.5 g, content: 49.7%, 0.78 mol) under a stream of nitrogen, which was washed with tetrahydrofuran (134 mL). To the mixture solution, a lithium hexamethyldisilazide tetrahydrofuran solution (1.0 M: 835.0 g, 0.94 mol) was added dropwise over 1.5 hours at -14°C at the inner temperature of not higher than - 10°C. Then, the reaction solution was stirred for 2 hours. After 10% aqueous solution of ammonium chloride (2818 g) was added, the aqueous layer was separated. The organic layer was washed sequentially with 5% sodium bicarbonate water (2818 g), 5% brine (1409 g), and water (1409 mL), and the solvent was removed under reduced pressure. To the residue, toluene was added, and azeotropic concentration was carried out under reduced pressure. The resultant concentrated product was diluted with toluene (564 mL) to give a solution of a crude product of the title compound in toluene (1360.0 g, content: 31.9%). Yield: 93.9%.

### 12) Synthesis of tert-butyl (3S)-3-[4-(benzyloxy)butyl]-5-(3,4,5-trifluorophenyl)-2,3-dihydro-4H-1,4-oxazine-4-carboxylate

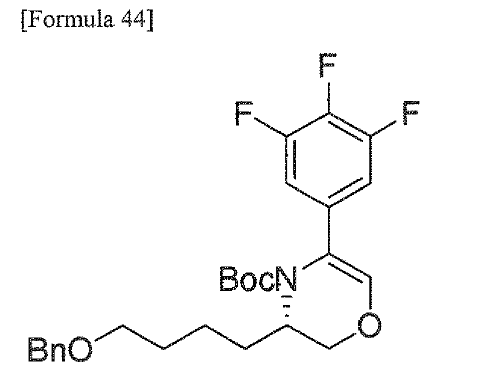

N,N-Dimethylformamide (719 mL), water (25.6 mL, 1.42 mol) and toluene (379.2 mL) were added to a solution of tert-butyl (3S)-3-[4-(benzyloxy)butyl]-5-[(diphenoxyphosphoryl)oxy]-2,3-dihydro-4H-1,4-oxazine-4-carboxylate in N,N-dimethylformamide (1880.7 g, content: 22.5%, 0.71 mol) obtained in the above 11). To the solution, 3,4,5-trifluorophenylboronic acid (187.4 g, 1.07 mol) was added, which was subjected to nitrogen substitution under reduced pressure. Then, bis dichloride(triphenylphosphine) palladium (24.96 g, 0.036 mol) and cesium carbonate (347.1 g, 1.07 mol) were added to the solution, which was subjected to nitrogen substitution again under reduced pressure. The reaction solution was stirred at the inner temperature of 80.0°C to 85.5°C for 2 hours and then cooled to the inner temperature of 25°C, and 50% aqueous solution of methanol (4789 mL) was added. To the reaction solution, n-heptane (3384 mL) was added at the inner temperature of 27°C. The mixture was filtered through Hyflo super-cel (Trade Mark) (Wako Pure Chemical Industries Ltd.) (431 g), and the filtrate was rinsed with n-heptane (1692 mL) and separated. The organic layer was washed twice with a 50% aqueous solution of methanol (4789 mL), and washed with 5% brine (2115 g) and water (2115 mL), sequentially. The organic layer was allowed to stand overnight, and then filtered through Hyflo super-cel (Trade Mark) (212 g) and washed with n-heptane (846 mL). The filtrate was evaporated to remove the solvent under reduced pressure, and substitution concentrated with methanol under reduced pressure. Then, the residue was dissolved in methanol (636 mL) to give a solution of a crude product of the title compound in methanol (855.0 g, content: 42.6%). Yield: >99%.

### 13) Synthesis of tert-butyl (3S,5R)-3-(4-hydroxybutyl)-5-(3,4,5-trifluorophenyl)-morpholine-4-carboxylate

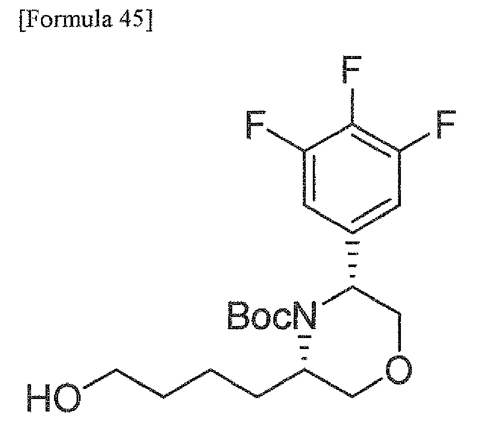

A solution of a crude product of tert-butyl (3S)-3-[4-(benzyloxy)butyl]-5-(3,4,5-trifluorophenyl)-2,3-dihydro-4H-1,4-oxazine-4-carboxylate in methanol (842.2 g, 0.75 mol) was placed in an autoclave and washed with methanol (200 mL). To the solution, 20% palladium hydroxide-C (50% water content, 71.7 g) was added, and the solution was washed with methanol (100 mL). To the mixture, methanol (732 mL) was added. After a reaction system was substituted with nitrogen and hydrogen, the mixture was stirred for 3 hours in a state in which the jacket temperature was set to 40°C under hydrogen pressure (0.20 MPa). The reaction mixture was filtered through Hyflo super-cel (Trade Mark), and the inside of the autoclave and the cake were washed with methanol (2152 mL). The filtrate was transferred to a reactor vessel with methanol (600 mL), and concentrated under reduced pressure. Then, the residue was washed out with methanol (600 mL) (1048.7 g). After the reaction solution was transferred to an autoclave with methanol (321 mL), 20% palladium hydroxide-C (50% water content, 35.9 g) was added, which was washed with methanol (200 mL). To the mixture, methanol (400 mL) was further added. After the inside of the reaction system was substituted with nitrogen and hydrogen, the mixture was stirred at the inner temperature of 40°C for 3 hours under hydrogen pressure (0.20 MPa), and further stirred at the inner temperature of 50°C for 12 hours. After the reaction mixture was filtered through Hyflo super-cel (Trade Mark), and the inside of the autoclave and the cake were washed with methanol (1793 mL). The filtrate was transferred to a reactor vessel with methanol (500 mL), concentrated under reduced pressure, and substitution concentrated with acetonitrile under reduced pressure. The residue was diluted with and dissolved in acetonitrile (100 mL) and methanol (100 mL) to give an acetonitrile/methanol solution of the title compound (779.9 g, content: 30.8%) as a brown oil. Yield: 82.1%.

### 14) Synthesis of 4-[(3S,5R)-4-(tert-butoxycarbonyl)-5-(3,4,5-trifluorophenyl)morpholin-3-yl]butanoic acid

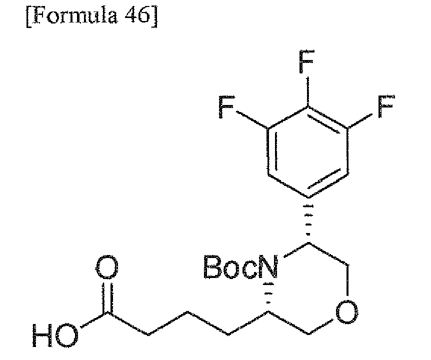

Acetonitrile (644 mL), water (1175 mL), sodium hydrogen carbonate (50.8 g, 0.60 mol), and (2,2,6,6-tetramethylpiperidine 1-oxyl (TEMPO) (9.4 g, 0.06 mol) were sequentially added to a suspension of a crude product of tert-butyl (3S,5R)-3-(4-hydroxybutyl)-5-(3,4,5-trifluorophenyl)morpholine-4-carboxylate in acetonitrile (650 g, content: 36.2%, 0.60 mol) with stirring under nitrogen atmosphere. The mixture was cooled to 5°C. To the mixture, an aqueous solution of sodium hypochlorite (detail: available chlorine content: 11.96%, 501 mL; available chlorine content: 10.95%, 479 mL; 1.81 mol) was added dropwise at the inner temperature of 20°C or below. After stirring for one hour, to the reaction solution, toluene (1175 mL) was added, and furthermore, 10% aqueous solution of sodium sulfite (1175 mL) was added dropwise. The reaction mixture was warmed up to 20°C and stirred for 30 minutes, and then 5 N aqueous hydrochloric acid (353 mL) was added dropwise thereto, and the aqueous layer was separated. To the organic layer, 1 N aqueous solution of sodium hydroxide (1880 mL) was added with stirring. Furthermore, the organic layer was treated with a 1 N aqueous solution of sodium hydroxide (470 mL). Then, the organic layer was separated. To the combined aqueous layers, 5 N aqueous hydrochloric acid (470 mL) was added, followed by extraction with ethyl acetate (2350 mL). The organic layer was washed sequentially with 5% brine (1175 mL) and water (1175 mL), the solvent was removed by evaporation under reduced pressure, and then substitution concentration with dimethoxyethane was carried out under reduced pressure. The residue was diluted with 1, 2-dimethoxyethane (118 mL) to give a solution of a crude product of the title compound in 1,2-dimethoxyethane (554.7 g, content: 43.5%). Yield: 99.2%.

### 15) Synthesis of 4-[(3S,5R)-5-(3,4,5-trifluorophenyl)morpholin-3-yl]butanoic acid monohydrochloride

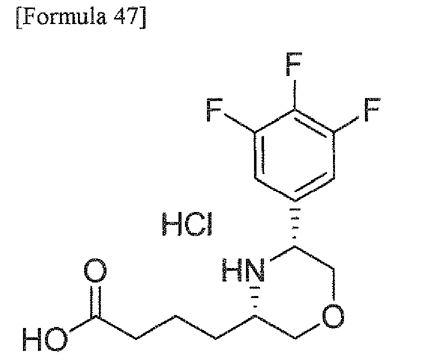

1,2-Dimethoxyethane (304 mL) was added to a solution of a crude product of 4-[(3S,5R)-4-(tert-butoxycarbonyl)-5-(3,4,5-trifluorophenyl)morpholin-3-yl]butanoic acid in 1,2-dimethoxyethane (548.4 g, content: 43.5%; 0.59 mol) under nitrogen atmosphere. To the solution, concentrated hydrochloric acid (95 mL, 1.06 mol) was added with stirring, and the solution was washed with 1,2-dimethoxyethane (54 mL). The mixture was heated at 50°C for 4 hours, and the reaction solution was cooled to 25°C. Then, to a suspension of precipitated crystals, tert-butyl methyl ether (715 mL) was added dropwise over 30 minutes. The suspension was stirred at the same temperature for one hour. The suspension was stirred at 15°C for 10 hours. Crystals were filtered out and washed with a tert-butyl methyl ether/1,2-dimethoxyethane mixture solution (715 mL). The resultant wet crystals were dried under reduced pressure at 45°C to give the title compound (184.0 g) as white crystals. Yield: 91.8%.

### 16) Synthesis of (4R,9aS)-4-(3,4,5-trifluorophenyl)hexahydropyrido[2,1-c][1,4]oxazin-6(1H)-one

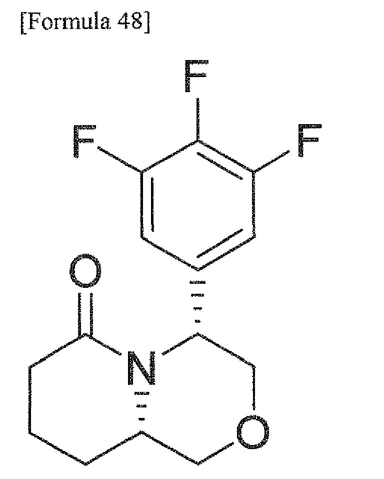

### i) Intramolecular condensation reaction

Isopropyl acetate (1800 mL) and acetonitrile (855 mL) were added to 4-[(3S,5R)-5-(3,4,5-trifluorophenyl)morpholin-3-yl]butanoic acid monohydrochloride (180 g, 0.53 mol) under a stream of nitrogen. The resultant suspension was cooled to about 16°C while triethylamine (184.4 mL, 1.33 mol) was added dropwise to the suspension with stirring, which was washed with acetonitrile (22.5 mL). To the reaction solution, isobutyl chloroformate (82.5 mL, 0.64 mol) was added dropwise at 174°C, and the reaction solution was washed with acetonitrile (22.5 mL). The reaction solution was stirred at the same temperature for one hour. Then, water (1800 mL) was added and the organic layer was extracted. Then, the organic layer was washed with 1 N aqueous hydrochloric acid (900 mL), 5% sodium bicarbonate water (900 g), and water (900 mL), sequentially. The solvent was removed by evaporation under reduced pressure, azeotropic concentration with isopropyl acetate was carried out under reduced pressure, and the residue was diluted with isopropyl acetate (400 mL) to give a solution of a crude product of the title compound in isopropyl acetate (872.1 g, content: 15.8%). Yield: 91.2%.

### ii) Preparation of seed crystal

The solution of the crude product of (4R,9aS)-4-(3,4,5-trifluorophenyl)hexahydropyrido[2,1-c][1,4]oxazin-6(1H)-one in isopropyl acetate, which was obtained in the above i) (6.8 g, content: 15.8%; 3.75 mmol) was concentrated under reduced pressure. The residue was dissolved in tert-butyl acetate (4 mL). The mixture was warmed to 40°C with stirring, and then cooled so that the inner temperature was 0°C so as to precipitate crystals. To the suspension, n-heptane (20 mL) was added dropwise over about 20 minutes. After the suspension was stirred at the same temperature for 30 minutes, precipitated crystals were filtered out and rinsed with n-heptane (5 mL). The wet crystals were dried under reduced pressure at 45°C to give crystals of the title compound (884.3 mg). The crystals were dissolved in tert-butyl acetate (3.5 mL) again at 40°C, and then the mixture was gradually cooled to 10°C over one hour to precipitate crystals and stirred for 11 hours after the outer temperature was set to 5°C. After n-heptane (17 mL) was added dropwise over 20 minutes, the mixture was stirred for 15 minutes in a state in which the outer temperature was set to -5°C. Precipitated crystals were filtered out, washed with n-heptane (5 mL), and dried under reduced pressure at 45°C to give the target seed crystals of the title compound (772.4 mg) as white crystals. Yield: 72.1%.

### iii) Crystallization purification

The solution of the crude product of (4R,9aS)-4-(3,4,5-trifluorophenyl)hexahydropyrido[2,1-c][1,4]oxazin-6(1H)-one in isopropyl acetate, which was obtained in the above i) (863.8 g, content: 15.8%; 0.48 mol) was concentrated under reduced pressure. The residue was dissolved in tert-butyl acetate (546 mL). The mixture was warmed to 43°C with stirring, then cooled so that the inner temperature was 20°C, seed crystals (137 mg) prepared in the iii) were added, and the mixture was gradually cooled at the cooling rate of 10°C/h so that the inner temperature was 2°C. After 17 hours from the start of gradual cooling, n-heptane (2730 mL) was added dropwise over 4.5 hours. The mixture was stirred at the same temperature for one hour. Furthermore, the mixture was gradually cooled to -13°C at the cooling rate of 10°C/h, and further stirred at the same temperature for 16 hours. Precipitated crystals were filtered out, then washed with n-heptane, and dried under reduced pressure at 45°C to give the title compound (120.8 g). Yield: 88.5%. Optical purity: 99.7%ee.

### INDUSTRIAL APPLICABILITY

The present invention provides a process for production of 4-(substituted phenyl)hexahydropyrido[2,1-c][1,4]oxazin-6-one useful as an intermediate product of a bicyclic cinnamide compound that is an Aβ production inhibitor. Furthermore, the present invention also provides a new compound used in the production process.

## Claims

1. A process for production of 4-(substituted phenyl)hexahydropyrido[2,1-c][1,4]oxazin-6-one or a salt thereof by subjecting a compound represented by the following formula (II-a): 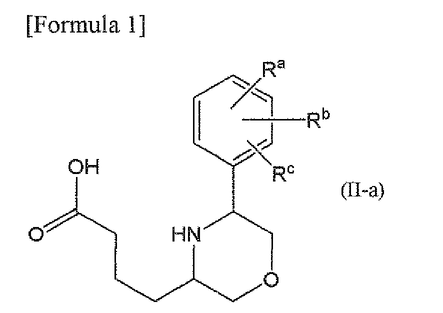 wherein R^{a}, R^{b} and R^{c} each independently represent a hydrogen atom, a halogen atom, a cyano group, an amino group, a nitro group, a C1-6 alkyl group or a C1-6 alkoxy group, or a salt thereof to an intramolecular condensation reaction, thereby producing a compound represented by the following formula (I-a): 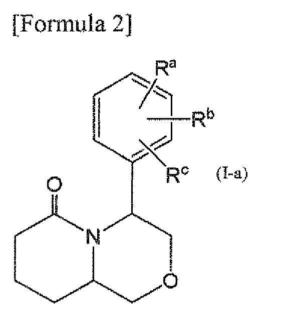 wherein R^{a}, R^{b} and R^{c} are as defined above, or a salt thereof.

2. The production process according to claim 7, wherein R^{a}, R^{b} and R^{c} are each independently a hydrogen atom or a halogen atom.

3. A process for production of 4-(3,4,5-trifluorophenyl)hexahydropyrido[2,1-c][1,4]oxazin-6-one or a salt thereof by subjecting a compound represented by the following formula (II): 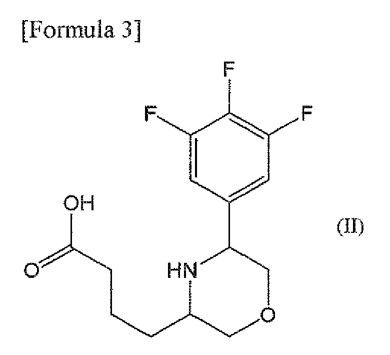 or a salt thereof to an intramolecular condensation reaction, thereby producing a compound represented by the following formula (I): 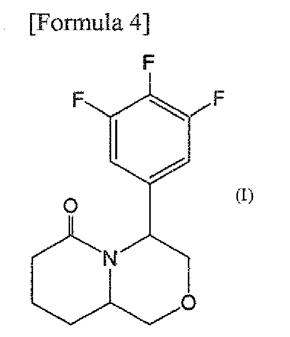 or a salt thereof.

4. A process for production of (4S,9aR)-4-(3,4,5-trifluorophenyl)hexahydropyrido[2,1-c][1,4]oxazin-6-one or a salt thereof by subjecting a compound represented by the following formula (II-b): 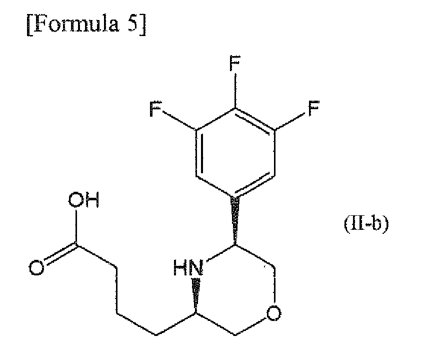 or a salt thereof to an intramolecular condensation reaction, thereby producing a compound represented by the following formula (I-b): 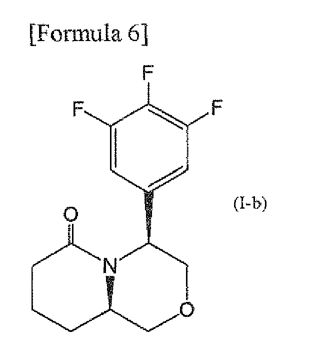 or a salt thereof

5. A process for production of (4R,9aS)-4-(3,4,5-trifluorophenyl) hexahydropyrido[2,1-c][1,4]oxazin-6-one or a salt thereof by subjecting a compound represented by a formula (II-c): 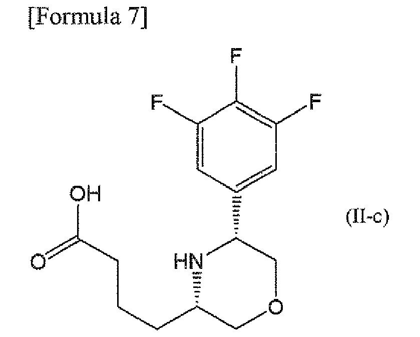 or a salt thereof to an intramolecular condensation reaction, thereby producing a compound represented by the following formula (I-c): 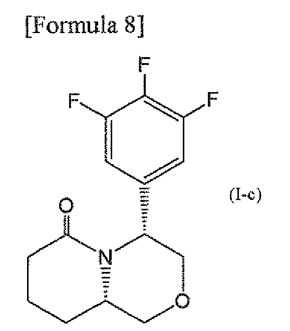 or a salt thereof.

6. A compound represented by the following formula (II-a): 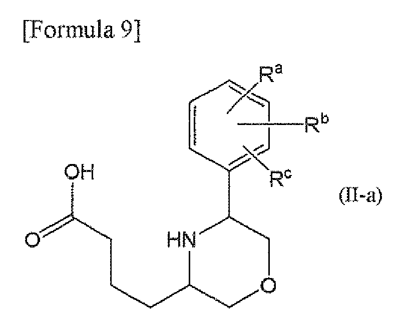 wherein R^{a}, R^{b} and R^{c} each independently represent a hydrogen atom, a halogen atom, a cyano group, an amino group, a nitro group, a C1-6 alkyl group or a C1-6 alkoxy group, or a salt thereof.

7. A compound represented by the following formula (II): 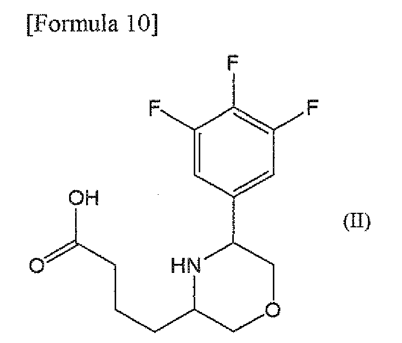 or a salt thereof.

8. A compound represented by the following formula (II-b): 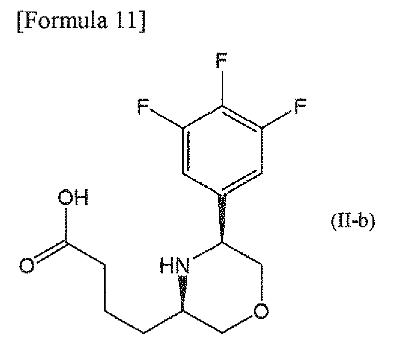 or a salt thereof.

9. A compound represented by the following formula (II-c): 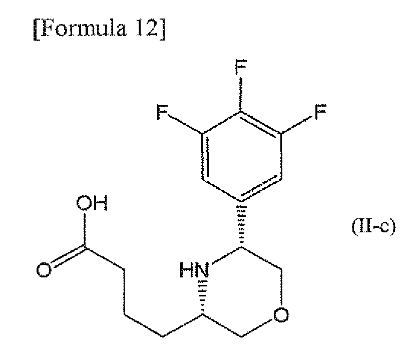 or a salt thereof.
